# EUROPEAN PATENT SPECIFICATION

(11) **EP 1 863 834 B1**
(45) Date of publication and mention of the grant of the patent: **14.07.2010**
(21) Application number: 06706131.7
(22) Date of filing: 22.03.2006
(51) Int. Cl.: C07K 14/32, C12N 9/00, C12N 15/31, C12N 15/52, A23K 1/00

(54) **POLYPEPTIDES AND NUCLEIC ACIDS ENCODING SAME**
POLYPEPTIDE UND NUKLEINSÄUREN, DIE DIESE CODIEREN
POLYPEPTIDES ET ACIDES NUCLÉIQUES LES CODANT

(30) Priority: 22.03.2005 DK 200500413; 29.06.2005 DK 200500966
(43) Date of publication of application: 12.12.2007
(73) Proprietor: Novozymes A/S, 2880 Bagsvaerd (DK)
(72) Inventor: OESTERGAARD, Peter, Rahbek, DK-2830 Virum (DK); ANDERSEN, Lene, Nonboe, DK-3450 Allerød (DK); SEON, Aurelia, F-68100 Mulhouse (FR); SIMOES-NUNES, Carlos, F-68128 Village-Neuf (FR); KLÜNTER, Anna-Maria, D-79541 Lörrach (DE); DE MARIA, Leonardo, DK-2000 Frederiksberg (DK); CHRISTENSEN, Bjørn, Eggert, DK-2880 Bagsværd (DK)
(86) International application number: PCT/DK2006/000162
(87) International publication number: WO 2006/099871

(56) References cited:
- WO- -02/29113
- WO-A-96/05739
- WO-A-03/093453
- WO-A-2004/095939
- WO-A2-02/29113
- GB-A- 2 138 023
- DATABASE Geneseq [Online] 27 February 2002 (2002-02-27), "Propionibacterium acnes immunogenic protein #4891." XP002386484 retrieved from EBI accession no. GSP:AAU43995 Database accession no. AAU43995 & WO 01/81581 A (CORIXA CORPORATION; SKEIKY, YASIR, A., W; PERSING, DAVID, H; MITCHAM,) 1 November 2001 (2001-11-01)
- ALEXOPOULOS C ET AL: "Field evaluation of the effect of a probiotic-containing Bacillus licheniformis and Bacillus subtilis spores on the health status, performance, and carcass quality of grower and finisher pigs" JOURNAL OF VETERINARY MEDICINE SERIES A, vol. 51, no. 6, August 2004 (2004-08), pages 306-312, XP002386483 ISSN: 0931-184X
- DATABASE WPI Section Ch, Week 200169 Derwent Publications Ltd., London, GB; Class B04, AN 2001-606161 XP002386500 & RU 2 172 343 C2 (VECTOR VIROLOGY & BIOTECHN RES CENTRE) 20 August 2001 (2001-08-20)
- DATABASE MEDLINE [Online] US NATIONAL LIBRARY OF MEDICINE (NLM), BETHESDA, MD, US; October 1995 (1995-10), RADA V ET AL: "[The effect of Lactobacillus salivarius administration on coliform bacteria and enterococci in the crop and cecum of broiler chickens]" XP002386485 Database accession no. NLM8659080 & VETERINÁRNÍ MEDICÍNA. OCT 1995, vol. 40, no. 10, October 1995 (1995-10), pages 311-315, ISSN: 0375-8427
- DATABASE UniProt [Online] 25 October 2004 (2004-10-25), "Hypothetical protein." XP002361793 retrieved from EBI accession no. UNIPROT:Q65CU4 Database accession no. Q65CU4 & VEITH B ET AL: "THE COMPLETE GENOME SEQUENCE OF BACILLUS LICHENIFORMIS DSM13, AN ORGANISM WITH GREAT INDUSTRIAL POTENTIAL" JOURNAL OF MOLECULAR MICROBIOLOGY AND BIOTECHNOLOGY, HORIZON SCIENTIFIC PRESS, WYMONDHAM,, GB, vol. 7, no. 4, 2004, pages 204-211, XP009047713 ISSN: 1464-1801 & REY MICHAEL W ET AL: "Complete genome sequence of the industrial bacterium Bacillus licheniformis and comparisons with closely related Bacillus species." GENOME BIOLOGY 2004, vol. 5, no. 10, 2004, page R77, XP002362812 ISSN: 1465-6914

## Description

### Field of the Invention

The present invention relates to the non-therapeutic use of certain isolated polypeptides in animal feed, e.g. for improving the Feed Conversion Ratio (FCR) and/or for modulation of the gut microflora. An example of a polypeptide of the invention is the so-called L12 protein from Bacillus licheniformis ATCC 14580, which has the amino acid sequence of amino acids +1 to +85 of SEQ ID NO:2 herein (in what follows amino acids 1-85 of SEQ ID NO:2). The invention also relates to the non-therapeutic probiotic use in animal feed of strains of Bacillus which produce proteins related to L12.

### Background of the Invention

### Background Art

WO 960739 discloses the use of a combination of (i) a xylanase, (ii) a protease, and optionally a β-glucanase in feed additives. In the additive the ratio of xylanase activity per g feed additive to the β-glucanase activity per g feed additive is 1:0-0.25. In WO 960739 it is stated that the inclusion of this enzyme feed additive improves the digestion of the animal.

WO 03/093453 discloses, in Example 1, the design of an improved Bacillus licheniformis host cell which does not produce a small extracellular protein encoded by nucleotides 601 to 978 of SEQ ID NO:133 of WO 03/093453, the protein having the amino acid sequence of amino acids 1-126 of SEQ ID NO:134 of WO 03/093453. SEQ ID NOs 133 and 134 of WO 03/093453 are included in the present sequence listing as SEQ ID NOs 3 and 4, respectively. The sequence of amino acids 1-126 of SEQ ID NO:4 herein is identical to amino acids -41 to +85 of SEQ ID NO:2 herein. WO 03/093453 does not disclose an isolated polypeptide having amino acids 1-85 of SEQ ID NO:2. WO 03/093453 does also not disclose an isolated nucleic acid sequence having nucleotides 124-378 of SEQ ID NO:1.

GenPept accession no. YP_081375 is a hypothetical protein BL00275 from Bacillus licheniformis ATCC 14580. GenPept accession no. YP_081375 is identical to amino acids -41 to +85 of SEQ ID NO:2 herein. The sequence of the hypothetical protein BL00275 from Bacillus licheniformis ATCC 14580 has also been entered in UniProtKB/TrEMBL with the primary accession number Q65CU4. This sequence is also identical to amino acids -41 to +85 of SEQ ID NO:2 herein.

The nucleotide sequence encoding YP_081375 has GenBank accession no. NC_006270. GenBank accession no. NC_006270 is identical to nucleotides 1-381 of SEQ ID NO:1 herein.

The present inventors surprisingly found that polypeptides related to part of this Bacillus licheniformis hypothetical sequence, viz. amino acids 1-85 of SEQ ID NO:2, have a great potential for use in animal feed.

SWISSPROT accession no. Q8DQM5 is a 115 amino acids hypothetical protein spr0600 from Streptococcus pneumoniae (strain ATCC BAA-255 / R6). The sequence is also disclosed in "Genome of the bacterium Streptococcus pneumoniae strain R6" by Hoskins et al, J. Bacteriol. 183:5709 (2001). Q8DQM5 does not comprise an amino acid sequence which has at least 33% identity to amino acids 1-85 of SEQ ID NO:2.

Martinez et al, Microbiology (1999), vol. 145, pp. 3155-3161 disclose, in Fig. 3, the deduced 91 amino acids sequence of the pre-bacteriocin lactococcin 972 with a predicted 66 amino acids C-terminal mature portion. The percentage of identity of each of the pre-polypeptide and the mature polypeptide to amino acids 1-85 of SEQ ID NO:2 is below 33%.

A search of nucleotide databases with nucleotides 124-378 of SEQ ID NO:1 (which encodes amino acids 1-85 of SEQ ID NO:2) revealed a DNA fragment of 47% identity to nucleotides 124-378 of SEQ ID NO:1. This fragment is part of a sequence resulting from a still on-going sequencing project. The organism from which the fragment derives is called Bacillus (Geobacillus) stearothermophilus strain 10. The sequencing work is done at the University of Oklahoma (http://www.genome.ou.edu/.bstearo.html). There is no publication of any of the amino acid sequences potentially corresponding to this DNA fragment, and therefore also no indication of any potential utility of any encoded amino acid sequence.

An in-feed probiotic product designated BioPlus™2B is offered for sale by Chr. Hansen A/S, 10-12 Boege Allé, DK-2970 Hoersholm, Denmark. The product contains a strain of Bacillus licheniformis which however does not produce a protein related to L12 (see Example 6 herein).

It is an object of the present invention to provide special strains of Bacillus, as well asnon-therapeutic methods for the use of the polypeptides and the Bacillus strains in animal feed.

### Summary of the Invention

The present invention relates to the non-therapeutic use of isolated polypeptides selected from the group consisting of: (a) a polypeptide having amino acids 1-126 of SEQ ID NO:4; (b) a polypeptide comprising an amino acid sequence which has a degree of identity to amino acids 1-85 of SEQ ID NO:2 of at least 33%; as determined by the Needle program, using the BLOSUM62 substitution matrix, a gap opening penalty of 10, and a gap extension penalty of 0.5; and (c) a polypeptide which is encoded by a nucleic acid sequence which hybridizes under medium stringency conditions with (i) nucleotides 124-378 of SEQ ID NO:1, (ii) a subsequence of (i) of at least 100 nucleotides, or (iii) a complementary strand of (i), or (ii); (c) within animal feed.

The present invention also relates to nucleic acid constructs, vectors, and host cells comprising the nucleic acid sequences as well as methods for producing and using the polypeptides within animal feed.

The present invention furthermore relates to the use in animal feed of a strain of Bacillus which is positive in the test of Example 6 herein, viz. the DNA of which, when harvested and used as a DNA template in a PCR reaction with SEQ ID NOs:6 and 7 as primers, leads to the generation of a PCR fragment of a size of approximately 0.4kb.

### Detailed Description of the Invention

### The Polypeptide, its Characteristics, and Use

The invention relates to the non-therapeutic use in animal feed of a polypeptide selected from the group consisting of:
(a) a polypeptide having amino acids 1-126 of SEQ ID NO:4;
(b) a polypeptide comprising an amino acid sequence which has a degree of identity to amino acids 1-85 of SEQ ID NO:2 of at least 33%, as determined by the Needle program, using the BLOSUM62 substitution matrix, a gap opening penalty of 10, and a gap extension penalty of 0.5; and
(c) a polypeptide which is encoded by a nucleic acid sequence which hybridizes under medium stringency conditions with (i) nucleotides 124-378 of SEQ ID NO:1, (ii) a subsequence of (i) of at least 100 nucleotides, and/or (iii) a complementary strand of (i), or (ii) as well as the use of any of these in the preparation of a composition for use in animal feed. These polypeptides improve animal feed utilization by improving the feed conversion ratio (FCR), and/or modulating the gut microflora.

A polypeptide to be used in the present invention may be a bacterial or a fungal polypeptide. In a second particular embodiment, the polypeptide is a Gram positive bacterial polypeptide such as a Bacillus polypeptide or a variant thereof, for example a Bacillus licheniformis polypeptide e.g. derived from Bacillus licheniformis ATCC 14580, which is the type strain of Bacillus licheniformis and available on request from the American Type Culture Collection, ATCC. Preferred strains of Bacillus licheniformis are positive in the test of Example 6 herein, such as the following strains of Bacillus licheniformis: ATCC 14580 (=NCIB 9375), NCIMB 6346 (=DSM 8785), NCTC 1024, NCTC 1025, NCTC 2120, NCTC 7589, NCTC 9932, ATCC 21424, NCIMB 10689, and ATCC 53757.

The polypeptide improves animal feed utilization by improving the feed conversion ratio (FCR), and/or modulating the gut microflora. In alternative embodiments, the polypeptide improves animal feed digestibility, and/or maintains animal health by aiding in proper digestion and/or supporting immune system function.

The FCR may be determined on the basis of a broiler chicken growth trial comprising a first treatment in which the polypeptide is added to the animal feed in a concentration of 5 mg per kg feed, and a second treatment (control) with no addition of the polypeptide to the animal feed, each treatment consisting of 8 groups of 6 male chicken, the chicken being fed pellets of a maize/SBM48 diet ad libitum, the FCR being calculated as the feed intake in g/bird relative to the weight gain in g/bird for day 8-29 of the trial, the FCR for the first treatment being improved relative to the FCR of the second treatment. In a particular embodiment the growth trial is a cage trial. For further details, see Example 5.

The FCR may also be determined on the basis of a broiler chicken growth trial comprising a first treatment in which the polypeptide is added to the animal feed in a concentration of (i) 2.5, (ii) 5.0, or (iii) 7.5 mg per kg feed, and a second treatment (control) with no addition of the polypeptide to the animal feed, each treatment consisting of 10 groups of 6 male chicken, the chicken being fed pellets of a maize/SBM48 diet ad libitum, the FCR being calculated as the feed intake in g/bird relative to the weight gain in g/bird for day 8-29 of the trial, the FCR for the first treatment being improved relative to the FCR of the second treatment. In a particular embodiment the growth trial is a cage trial. For further details, see Example 9.

The FCR may also be determined on the basis of a broiler chicken growth trial comprising a first treatment in which the polypeptide is added to the animal feed in a concentration of 7.5 mg per kg feed, and a second treatment (control) with no addition of the polypeptide to the animal feed, each treatment consisting of 12 groups of 20 chicken (6 groups of 20 females, 6 groups of 20 males), the chicken being fed pellets of a diet based on wheat, rye and soybean meal ad libitum, the FCR being calculated as the feed intake in g/bird relative to the weight gain in g/bird for day 1-36 of the trial, the FCR for the first treatment being improved relative to the FCR of the second treatment. In a first particular embodiment the chicken are housed in floor pens littered with wood shavings. In a second particular embodiment the chicken are fed a starter diet in the period of day 1-22 and a grower diet in the period of day 22-36, both based on wheat, rye and soybean meal, and with a composition as shown in Table 9. For further details, see Example 12.

As it is generally known, an improved FCR is lower than the control FCR. In particular embodiments, the FCR is improved (i.e., reduced) as compared to the control by at least 1.0%, preferably at least 1.5%, 1.6%, 1.7%, 1.8%, 1.9%, 2.0%, 2.1%, 2.2%, 2.3%, 2.4%, or at least 2.5%. In further particular embodiments, the FCR is improved (i.e. reduced) as compared to the control by at least 2.6%, 2.7%, 2.8%, 2.9%, or at least 3.0%. In still further particular embodiments, the FCR is improved (i.e., reduced) as compared to the control by at least 3.1%, 3.2%, 3.3%, 3.4%, 3.5%, 3.6%, 3.7%, or at least 3.8%.

The term "gut" as used herein designates the gastrointestinal or digestive tract (also referred to as the alimentary canal) and it refers to the system of organs within multicellular animals which takes in food, digests it to extract energy and nutrients, and expels the remaining waste. The gut may be divided in upper and lower gastrointestinal tract, the former comprising the mouth and the stomach, and the latter comprising the intestines. The intestines may be divided in small and large intestine. The basic components of the small intestine, however with differences between animal species, are duodenum, jejunum, and ileum. The basic components of the large intestine are caecum, colon and rectum (cloaca).

The term gut "microflora" as used herein refers to the natural microbial cultures residing in the gut and maintaining health by aiding in proper digestion and/or supporting immune system function.

The term "modulate" as used herein in connection with the gut microflora generally means to change, manipulate, alter, or adjust the function or status thereof in a healthy and normally functioning animal, i.e. a non-therapeutic use. The modulation is in response to the polypeptides and/or the Bacillus strains of the invention.

The following are non-limiting particular examples of the gut microflora modulation effect obtained by the polypeptide of the invention (changes as compared to a control without the polypeptide) - for further details, see Example 8:
(i) an increase in the number of total facultative anaerobic bacteria in vivo, for example in piglets and/or broilers, preferably determined after cultivation of ileo-rectal and/or caecal contents, respectively, on Brucella agar supplemented with 5% vol/vol sheep blood after incubation in an anaerobic cabinet at 37 °C for five days;
(ii) an increase in the number of Escherichia coli in vivo, for example in piglets and/or broilers, preferably determined after cultivation of ileo-rectal and/or caecal contents, respectively, on Coli-ID chromogenic media, aerobically, at 37°C for 24 hours;
(iii) no substantial change, or an increase, in total lactic acid bacteria in vivo, for example in piglets and/or broilers, preferably determined after cultivation of ileo-rectal and/or caecal contents, respectively, on MRS agar, in an anaerobic cabinet, at 37°C for 48 hours;
(iv) no substantial change in total Lactobacillus spp. in vivo, for example in piglets, preferably determined after cultivation of ileo-rectal contents on Rogosa agar, in an anaerobic cabinet at 37°C for 48 hours;
(v) a decrease in the number of other Enterobacteriaceae (other than E. coli) in vivo, for example in piglets, preferably determined after cultivation of ileo-rectal contents on a Coli-ID chromogenic media, aerobically, at 37°C for 24 hours;
(vi) a decrease in the number of Enterococcus spp. in vivo, for example in piglets, preferably determined after cultivation of ileo-rectal contents on an Enterococci agar, aerobically, at 37°C for 48 hours; and/or
(vii) a decrease in the frequency with which Clostridium perfringens occurs in vivo, for example in piglets, preferably determined after cultivation of ileo-rectal contents on TSN agar after heating the sample at 80°C during 10 min, in an anaerobic cabinet at 46°C for 24 hours.

Still further, also in relation to the gut microflora modulating effect, and with reference to a control without the polypeptide of the invention, the polypeptide preferably:
(iix) is more active in vitro against Gram positive cocci than Gram negative bacteria, viz. exhibiting a stronger reduction of the former, for example for Gram positive cocci and Gram negative bacteria isolated from piglet and/or broiler intestinal contents;
(ix) is more active in vitro against Clostridium spp., for example Clostridium perfringens, than Gram negative bacteria, viz. exhibiting a stronger reduction of the former, for example for Clostridium perfringens and Gram negative bacteria isolated from piglet and/or broiler intestinal contents;
(x) does not substantially influence the growth in vitro of beneficial microorganisms, such as bacteria, for example as isolated from piglet and/or broiler intestinal contents; and/or (xi) does substantially influence, e.g. reduce, the growth in vitro of harmful microorganisms, such as bacteria, for example as isolated from piglet and/or broiler intestinal contents.

For details in relation to embodiments (iix)-(xi), please see the last part of Example 8.

In embodiment (x), the beneficial microorganisms are preferably selected from Lactobacillus spp., such as Lactobacillus salivarius DSM 18070, for which the MIC₉₀ is at least 1000 microgram/ml, preferably at least 2000, 3000, 4000, 4500, 5000, 5500, 6000, or at least 7000 microgram/ml, in particular at least 4170 microgram/ml.

Accordingly, the polypeptide has a MIC₉₀ against Lactobacillus salivarius DSM 18070 of at least 1000 microgram/ml, preferably at least 2000, 3000, 4000, 4500, 5000, 5500, 6000, or at least 7000 microgram/ml, in particular at least 4170 microgram/ml.

In embodiment (xi), the harmful microorganisms are preferably selected from Enterococcus, Staphylococcus, Clostridium (preferably Clostridium perfringens); such as Enterococcus faecalis DSM 18047, for which the MIC₉₀ is below 4000 microgram/ml, preferably below 3000, 2000, 1000, 800, 600, 500, 400, 300, 200, 100, 90, 80, or below 70 microgram/ml, preferably a MIC₉₀ of 50-80, more preferably 60-70 microgram/ml.

Accordingly, the polypeptide has a MIC₉₀ against Enterococcus faecalis DSM 18047 below 4000 microgram/ml, preferably below 3000, 2000, 1000, 800, 600, 500, 400, 300, 200, 100, ,90, 80, or below 70 microgram/ml, preferably a MIC₉₀ of 50-80, more preferably 60-70 microgram/ml.

MIC₉₀ designates the Minimum Inhibitory Concentration required for inhibiting the growth of 90% of organisms. For the present purposes, MIC₉₀ is defined as that concentration of the polypeptide which is required to reduce culture density, determined as OD₅₉₅, by 90% relative to a control without the polypeptide. OD₅₉₅ is determined after incubation under appropriate incubation conditions (which depend on the organism in question, as shown in Table 5 of Example 8). The determination of MIC₉₀ may be made by a micro titre broth dilution method, using approximately 10⁵ CFU/ml of the pure bacteria in tryptic soy broth, adding the polypeptide in serial 2-fold dilutions, using water as control. For further details, see Example 8. MIC₉₀ is herein generally indicated in the unit of microgram of the pure polypeptide per ml (microgram/ml).

In still further particular embodiments, the polypeptide:
(xii) has a MIC₉₀ for a Gram negative bacterium, such as a strain of Escherichia coli, Klebsiella pneumoniae, Salmonella enteritidis, Salmonella typhimurium, or Proteus mirabilis, which is at least 50% higher than a MIC₉₀ for a Gram positive coccus, such as a strain of Enterococcus or Staphylococcus, e.g. Enterococcus faecalis, preferably Enterococcus faecalis DSM 18047 - in preferred sub-embodiments hereof, the MIC₉₀ of the former is preferably at least 100%, 150%, 200%, 250%, 300%, 350%, 400%, 450%, or at least 500% higher than the MIC₉₀ of the latter.

In a fourth particular embodiment (see Example 11 for details), the polypeptide in its native (i.e. non-denatured) form, is not degraded by (i) pepsin (pH 3.5, 40°C, 2 hours); (ii) pancreatin (pH 7.0, 40°C, 4 hours); and/or (iii) pepsin-followed-by-pancreatin (pepsin at pH 3.5, 40°C, 2 hours - followed by pancreatin at pH 7.0, 40°C, 4 hours) - for all embodiments (i)-(iii) as judged by SDS-PAGE (see Example 10).

In a fifth particular embodiment (see Example 7 for details), the polypeptide has the following denaturation temperatures, as determined by Differential Scanning Calorimetry (DSC): (i) at least 54°C at pH 2.5, (ii) at least 68°C at pH 4.0, and/or (iii) at least 59°C at pH 7.0; preferably (iv) 55°C at pH 2.5; (v) 69°C at pH 4.0; and/or (vi) 60°C at pH 7.0.

### Identity and Hybridization, Fragments and Variants

The relatedness between two amino acid sequences is described by the parameter "identity".

For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence to be used according to the present invention ("invention sequence"; e.g. amino acids 1-85 of SEQ ID NO:2, and a different amino acid sequence ("foreign sequence") is calculated as the number of exact matches in an alignment of the two sequences, divided by the length of the "invention sequence" or the length of the "foreign sequence", whichever is the shortest. The result is expressed in percent identity.

An exact match occurs when the "invention sequence" and the "foreign sequence" have identical amino acid residues in the same positions of the overlap (in the alignment example below this is represented by "I"). The length of a sequence is the number of amino acid residues in the sequence (e.g. the length of amino acids 1-85 of SEQ ID NO:2 is 85).

In the purely hypothetical alignment example below, the overlap is the amino acid sequence "HTWGER-NL" of Sequence 1; or the amino acid sequence "HGWGEDANL" of Sequence 2. In the example a gap is indicated by a "-".

Hypothetical alignment example:

The percentage of identity of an amino acid sequence of a polypeptide with, or to, amino acids 1-85 of SEQ ID NO:2 is determined by i) aligning the two amino acid sequences using the Needle program, with the BLOSUM62 substitution matrix, a gap opening penalty of 10, and a gap extension penalty of 0.5; ii) counting the number of exact matches in the alignment; iii) dividing the number of exact matches by the length of the shortest of the two amino acid sequences, and iv) converting the result of the division of iii) into percentage. In the above hypothetical example, the number of exact matches is 6, the length of the shortest of the two amino acid sequences is 12, accordingly the percentage of identity is 50%.

In the alternative, the degree of identity between two amino acid sequences, as well as the degree of identity between two nucleotide sequences, is determined by the program "align" which is a Needleman-Wunsch alignment (i.e. a global alignment). The program is used for alignment of polypeptide, as well as nucleotide sequences. The default scoring matrix BLOSUM50 is used for polypeptide alignments, and the default identity matrix is used for nucleotide alignments. The penalty for the first residue of a gap is -10 for polypeptides and -16 for nucleotides. The penalties for further residues of a gap are -2 for polypeptides, and -4 for nucleotides. "Align" is part of the FASTA package version v20u6 (see W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA," Methods in Enzymology 183:63-98). FASTA protein alignments use the Smith-Waterman algorithm with no limitation on gap size (see "Smith-Waterman algorithm", T. F. Smith and M. S. Waterman (1981) J. Mol. Biol. 147:195-197). See also Myers and Miller, CABIOS (1989) 4:11-17.

In preferred embodiments, the degree of identity to amino acids 1-85 of SEQ ID NO:2 is at least 35%, or a least 37%, 40%, 42%, 45%, 47%, 50%, 52%, 55%, 57%, 60%, 62%, 65%, 67%, 70%, 72%, 75%, 77%, 80%, 82%, 85%, 87%, 90%, 92%, 95%, 97%, or at least 99%. Polypeptides with any of these degrees of identity to amino acids 1-85 of SEQ ID NO:2 are referred to as homologous polypeptides. In an alternative embodiment, the degree of identity to amino acids 1-85 of SEQ ID NO:2 is 32%.

In particular embodiments, the polypeptides comprise (or have, or consist of) an amino acid sequence that differs by (i) 57, 55, 50, 45, 40, 35, 30, or 25 amino acids from amino acids 1-85 of SEQ ID NO:2; or by (ii) 20, 19, 18, 17, 16, 15, 14, 13, 12, or 11 amino acids from amino acids 1-85 of SEQ ID NO:2; or by (iii) 10, 9, 8, 7, 6, or 5 amino acids from amino acids 1-85 of SEQ ID NO:2. In a further particular embodiment, the polypeptides comprise (or have, or consist of) an amino acid sequence that differs by 4, 3, or 2 amino acids, or by 1 amino acid from amino acids 1-85 of SEQ ID NO:2.

A fragment of, e.g., amino acids 1-85 of SEQ ID NO:2 is a polypeptide having one or more amino acids deleted from the amino and/or carboxyl terminus of these amino acid sequences. In one embodiment a fragment contains at least 30, 35, 40, 45, 50, or at least 55 amino acids. In another embodiment a fragment contains at least 65 amino acid residues, or at least 70 amino acid residues, or at least 75 amino acid residues, or at least 80 amino acid residues, or at least 81 amino acid residues, or at least 82 amino acid residues, or at least 83 amino acid residues, or at least 84 amino acid residues.

An allelic variant denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

The present invention also relates to the use of isolated polypeptides which are encoded by nucleic acid sequences which hybridize under medium, or medium-high, or high, or very high stringency conditions with a nucleic acid probe which hybridizes under the same conditions with (i) nucleotides 124-378 of SEQ ID NO:1, (ii) a subsequence of (i), or (iii) a complementary strand of (i), or (ii) (J. Sambrook, E.F. Fritsch, and T.. Maniatis, 1989, Molecular Cloning, A Laboratory Manual, 2nd edition, Cold Spring Harbor, New York). In one particular embodiment the nucleic acid probe is selected from amongst the nucleic acid sequences of (i), (ii), or (iii) above.

The subsequence of nucleotides 124-378 of SEQ ID NO:1 may be at least 100 nucleotides, or in another embodiment at least 50, 150, or 200 nucleotides.

The nucleic acid sequence of nucleotides 124-378 of SEQ ID NO:1 or a subsequence thereof, as well as the amino acid sequence of amino acids 1-85 of SEQ ID NO:2 or a fragment thereof, may be used to design a nucleic acid probe to identify and clone DNA encoding polypeptides having a potential for use in animal feed from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 35 nucleotides in length. Longer probes can also be used. Both DNA and RNA probes can be used. The probes are typically labelled for detecting the corresponding gene (for example, with ³²P, ³H, ³⁵S, biotin, or avidin). Such probes are encompassed by the present invention.

Thus, a genomic DNA or cDNA library prepared from such other organisms may be screened for DNA that hybridizes with the probes described above and which encodes a polypeptide having the desired activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA which is homologous with SEQ ID NO:1 or a subsequence thereof, the carrier material is used in a Southern blot. For purposes of the present invention, hybridization indicates that the nucleic acid sequence hybridizes to a labelled nucleic acid probe corresponding to the nucleic acid sequence shown in SEQ ID NO:1, its complementary strand, or a subsequence thereof, under very low to very high stringency conditions. Molecules to which the nucleic acid probe hybridizes under these conditions are detected using X-ray film.

In a particular embodiment, the nucleic acid probe is a nucleic acid sequence which encodes amino acids 1-85 of SEQ ID NO:2, or subsequences thereof. In another embodiment, the nucleic acid probe is nucleotides 124-378 of SEQ ID NO:1 (the mature polypeptide coding region of SEQ ID NO:1).

For long probes of at least 100 nucleotides in length, very low to very high stringency conditions are defined as prehybridization and hybridization at 42°C in 5X SSPE, 0.3% SDS, 200 µg/ml sheared and denatured salmon sperm DNA, and either 25% formamide for very low and low stringencies, 35% formamide for medium and medium-high stringencies, or 50% formamide for high and very high stringencies, following standard Southern blotting procedures.

For long probes of at least 100 nucleotides in length, the carrier material is finally washed three times each for 15 minutes using 2 x SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For short probes about 15 nucleotides to about 70 nucleotides in length, stringency conditions are defined as prehybridization, hybridization, and washing post-hybridization at 5°C to 10°C below the calculated Tₘ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes about 15 nucleotides to about 70 nucleotides in length, the carrier material is washed once in 6X SSC plus 0.1% SDS for 15 minutes and twice each for 15 minutes using 6X SSC at 5°C to 10°C below the calculated Tₘ.

The present invention also relates to the use of variants of the polypeptide having an amino acid sequence of amino acids 1-85 of SEQ ID NO:2 comprising a substitution, deletion, and/or insertion of one or more amino acids.

The amino acid sequences of the variant polypeptides may differ from the amino acid sequence of amino acids 1-85 of SEQ ID NO:2 by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Accordingly, for example, the invention relates to a polypeptide having, or comprising, a sequence as set forth in SEQ ID NO:2, preferably the mature part thereof, wherein conservative amino acid substitutions comprise replacements, one for another, among the basic amino acids (arginine, lysine and histidine), among the acidic amino acids (glutamic acid and aspartic acid), among the polar amino acids (glutamine and asparagine), among the hydrophobic amino acids (alanine, leucine, isoleucine, and valine), among the aromatic amino acids (phenylalanine, tryptophan and tyrosine), and among the small amino acids (glycine, alanine, serine, threonine and methionine), or any combination thereof, or active fragments thereof.

As defined herein, an "isolated" or "pure" polypeptide is a polypeptide which is essentially free of other polypeptides, e.g., at least 80% pure, preferably at least 85%, 86%, 87%, 88%, 89%, or at least 90% pure, more preferably at least 91%, 92%, 93%, 94%, 95%, or at least 96% pure, as determined by SDS-PAGE (e.g., by coomassie-staining and subsequent scanning by methods known in the art - see Examples 2 and 4). Purity may also be determined by HPLC, preferably RP-HPLC (e.g., using a Waters µ-Bondapak C18 column, Mobil phase A: 0.1% TFA, Mobil phase B: Acetonitrile + 0.1% TFA, detecting at 280nm - see Example 10). The SDS-PAGE purity, as well as the HPLC purity, refers to the amount of the polypeptide of the invention, relative to the amount of total protein. In alternative embodiments, the polypeptide may be at least 20%, 40%, 60%, or at least 70% pure.

The amount of total protein can be determined by any method known in the art, e.g. the Kjeldahl method (A.O.A.C., 1984, Official Methods of Analysis 14th ed., Association of Official Analytical Chemists, Washington DC), and the amount of the polypeptide of the invention can be determined by SDS-PAGE and subsequent scanning, also by methods known in the art.

Polypeptides encoded by nucleic acid sequences also include fused polypeptides or cleavable fusion polypeptides in which another polypeptide is fused at the N-terminus or the C-terminus of the polypeptide or fragment thereof. A fused polypeptide is produced by fusing a nucleic acid sequence (or a portion thereof) encoding another polypeptide to a nucleic acid sequence (or a portion thereof) encoding a polypeptide to be used according to the present invention. Techniques for producing fusion polypeptides are known in the art, and include ligating the coding sequences encoding the polypeptides so that they are in frame and that expression of the fused polypeptide is under control of the same promoter(s) and terminator.

In a specific embodiment, the polypeptide is a low-allergenic variant, designed to invoke a reduced immunological response when exposed to animals, including man. The term immunological response is to be understood as any reaction by the immune system of an animal exposed to the polypeptide. One type of immunological response is an allergic response leading to increased levels of IgE in the exposed animal. Low-allergenic variants may be prepared using techniques known in the art. For example the polypeptide may be conjugated with polymer moieties shielding portions or epitopes of the polypeptide involved in an immunological response. Conjugation with polymers may involve in vitro chemical coupling of polymer to the polypeptide, e.g. as described in WO 96/17929, WO98/30682, WO98/35026, and/or WO99/00489. Conjugation may in addition or alternatively thereto involve in vivo coupling of polymers to the polypeptide. Such conjugation may be achieved by genetic engineering of the nucleotide sequence encoding the polypeptide. Another way of providing low-allergenic variants is genetic engineering of the nucleotide sequence encoding the polypeptide so as to cause the polypeptides to self-oligomerize, effecting that polypeptide monomers may shield the epitopes of other polypeptide monomers and thereby lowering the antigenicity of the oligomers. Such products and their preparation is described e.g. in WO 96/16177. Epitopes involved in an immunological response may be identified by various methods such as the phage display method described in WO 00/26230 and WO 01/83559, or the random approach described in EP 561907. Once an epitope has been identified, its amino acid sequence may be altered to produce altered immunological properties of the polypeptide by known gene manipulation techniques such as site directed mutagenesis (see e.g. WO 00/26230, WO 00/26354 and/or WO 00/22103) and/or conjugation of a polymer may be done in sufficient proximity to the epitope for the polymer to shield the epitope.

### Bacterial strains; Probiotic Bacillus strains

The invention relates to the non-therapeutic use in animal feed of a strain of Bacillus, the DNA of which, when harvested and used as a DNA template in a PCR reaction with SEQ ID NOs:6 and 7 as primers, leads to the generation of a PCR fragment of a size of approximately 0.4kb. This test serves to identify strains with an L12-like gene, see Example 6 herein. These strains of Bacillus may also be used in the preparation of a composition for non-therapeutic use in animal feed. These uses are, e.g., with a view to improving the feed conversion ratio (FCR), and/or modulating the gut microflora.

In a first particular embodiment, the Bacillus strain is a probiotic microorganism. The term "probiotic" generally refers to a non-pathogenic bacterium fed to animals, including birds, as a way to prevent colonization by pathogenic bacteria. The basic concept is to encourage colonization of mucosal surfaces as a way of blocking colonization by serious pathogens. Probiotics may also be defined as live, or livable, micro-organisms which beneficially affect the intestinal balance of healthy and normally functioning humans and animals, thereby preferably increasing liveweight gain and/or improving feed conversion.

In a second particular embodiment, the Bacillus strain is used in the form of spores. Spores may be exospores or, preferably, endospores. An endospore is any spore that is produced within an organism (usually a bacterium). Endospores can survive through periods of environmental stress, and are therefore capable of surviving passage of the harsh (acid) environment of the upper gastro intestinal tract, while only exerting its effect once it reaches the intestines, where normal vegetative cells will be formed.

In a third particular embodiment, the PCR fragment, when purified and sequenced encodes an amino acid sequence which has at least 33% identity to amino acids 1-85 of SEQ ID NO:2. The particular embodiments of the first aspect of the invention (the non-therapeutic use in animal feed of the polypeptide ) are also applicable to this aspect of the invention.

In further particular embodiments, the strain of Bacillus is a strain of Bacillus licheniformis, preferably selected from the following strains of Bacillus licheniformis: ATCC 14580 (=NCIB 9375), NCIMB 6346 (=DSM 8785), NCTC 1024, NCTC 1025, NCTC 2120, NCTC 7589, NCTC 9932, ATCC 21424, NCIMB 10689, and ATCC 53757. A preferred subgroup includes Bacillus licheniformis ATCC 14580 (=NCIB 9375), and Bacillus licheniformis NCIMB 6346 (=DSM 8785).

In still further particular embodiments, the strain of Bacillus for use according to the invention is (i) not Bacillus licheniformis DSMZ 5749, (ii) not Bacillus subtilis DSMZ 5750, and/or (iii) not Bacillus licheniformis FERM BP-266. The strains of (i) and (ii) are included in the BioPlus™2B product, see, e.g., EP 1472933. The strain of (iii) is described in GB 2138023.

For a taxonomical classification and identification of bacteria reference is had to Bergey's Manual of Systematic Bacteriology (1986), vol 2, ISBN0-683-0783; see for example p 1104 section 13, Endospore forming Gram positive rods and cocci; p. 1105 Genus Bacillus; pp. 1105-1129 description of the genus; pp. 1130-1138 description of the individual Bacillus species, e.g. on p. 1132 Bacillus licheniformis). In the alternative, the well-known 16SrRNA sequence analysis can be used (see e.g. Johansen et al, Int. J. Syst. Bacteriol, 1999, 49, 1231-1240, in particular the Methods section on p. 1233, 2^{nd} column); or taxonomy experts can be consulted, e.g. from DSMZ or other recognized depositary institutes.

Strains of Bacillus, such as strains of Bacillus licheniformis, are known in the art and available from, e.g., culture collections like ATTC mentioned above, or they can be isolated from nature. Preparations of live, or livable, Bacillus cells may be prepared as is known in the art. Examples of such cells are vegetative cells, and spores such as endospores. In one embodiment a fermentation extract of the Bacillus strain is used, for example in the form of a spray dried fermentation liquor.

The test of Example 6 is a PCR reaction, in this example conducted with DNA isolated from various strains of Bacillus licheniformis. In a particular embodiment of this test, the DNA used as template for the PCR reaction is chromosomal DNA which can be isolated by methods known in the art. The result of the Example 6 test is positive when a PCR fragment of the right size is obtained. In example 6, the right size is indicated as 0.4kb. In a particular embodiment, the right size is between 0.35kb and 0.44kb (=350bp-440bp). In alternative embodiments, the right size is 330-430bp, 340-420bp, 350-410bp, 360-400bp, 370-390bp, or 385-395bp. The size of the coding sequence (CDS) of SEQ ID NO:1 is approximately 380bp (viz. 378bp).

### Nucleic Acid Sequences

The invention also relates to the non-therapeutic use of a polypeptide encoded by an isolated nucleic acid sequence comprising a polypeptide-encoding nucleic acid sequence, which hybridizes under medium stringency conditions with (i) nucleotides 124-378 of SEQ ID NO:1, (ii) a subsequence of (i) of at least 100 nucleotides, and/or (iii) a complementary strand of (i), or (ii).

Identity and hybridization is defined in a previous section.

In a first particular embodiment, the nucleic acid sequence encodes a polypeptide, the use of which improves animal feed utilization by improving the feed conversion ratio (FCR), and/or modulating the gut microflora.

A particular nucleic acid sequence is nucleotides 124-378 of SEQ ID NO:1, the latter corresponding to the mature polypeptide encoding region. Other particular nucleic acid sequences are those encoding the polypeptide of amino acids 1-85 of any one of SEQ ID NOs:2, 8, 9, and 10.

The present invention also encompasses the non-therapeutic use of fragments of SEQ ID NO:2 encoded by nucleic acid sequences which comprise a nucleic acid sequence encoding a polypeptide having the amino acid sequence of amino acids 1-85 of SEQ ID NO:2, which differ from the corresponding parts of SEQ ID NO:1 by virtue of the degeneracy of the genetic code. This includes subsequences of SEQ ID NO:1 which encode fragments of SEQ ID NO:2.

A subsequence of SEQ ID NO:1 is a nucleic acid sequence encompassed by SEQ ID NO:1 except that one or more nucleotides from the 5' and/or 3' end have been deleted. Preferably, a subsequence contains at least 150 nucleotides, more preferably at least 165, 180, 195, 210, 225, 240, 270, 285, 300, 315, 330, or most preferably at least 345 nucleotides.

The present invention also relates to the non-therapeutic use of polypeptides encoded by nucleotide sequences which comprise a nucleic acid sequence encoding a polypeptide and which has a degree of identity to nucleotides 124-378 of SEQ ID NO:1 of at least 48%. For determining the degree of nucleotide identity, the program "align" is used which is referred to above.

In preferred embodiments, the degree of identity to nucleotides 124-378 of SEQ ID NO:1 is at least 50%, 52%, 55%, 57%, 60%, 62%, 65%, 67%, 70%, 72%, 75%, 77%, 80%, 82%, 85%, 87%, 90%, 92%, 95%, 97%, or at least 99%. In alternative embodiments, the degree of identity to nucleotides 124-378 of SEQ ID NO:1 is at least 32%, or a least 35%, 37%, 40%, 42%, 45%, or at least 47%.

Such nucleotides also include mutant nucleic acid sequences comprising at least one mutation in nucleotides 124-378 of SEQ ID NO:1, in which the mutant nucleic acid sequence encodes a polypeptide which (i) consists of amino acids 1-85 of SEQ ID NO:2, or (ii) is a variant of the sequence of (i), wherein the variant comprises a substitution, deletion, and/or insertion of one or more amino acids, or (iii) is an allelic variant of the sequence of (i), or (iv) is a fragment of the sequence of (i).

The techniques used to isolate or clone a nucleic acid sequence encoding a polypeptide are known in the art and include isolation from genomic DNA, preparation from cDNA, or a combination thereof. The cloning of the nucleic acid sequences from such genomic DNA can be effected, e.g., by using the well known polymerase chain reaction (PCR) or antibody screening of expression libraries to detect cloned DNA fragments with shared structural features. See, e.g., Innis et al., 1990, PCR: A Guide to Methods and Application, Academic Press, New York. Other nucleic acid amplification procedures such as ligase chain reaction (LCR), ligated activated transcription (LAT) and nucleic acid sequence-based amplification (NASBA) may be used. The nucleic acid sequence may be cloned from a strain of Bacillus, preferably Bacillus licheniformis, or another or related organism and thus, for example, may be an allelic or species variant of the polypeptide encoding region of the nucleic acid sequence.

The term "isolated nucleic acid sequence" as used herein refers to a nucleic acid sequence which is essentially free of other nucleic acid sequences, e.g., at least about 20% pure, preferably at least about 40% pure, more preferably at least about 60% pure, even more preferably at least about 80% pure, and most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

Modification of a nucleic acid sequence encoding a polypeptide used in the present invention may be necessary for the synthesis of polypeptides substantially similar to the polypeptide. The term "substantially similar" to the polypeptide refers to non-naturally occurring forms of the polypeptide. These polypeptides may differ in some engineered way from the polypeptide isolated from its native source, e.g., variants that differ in thermostability, pH stability, stability towards digestive enzymes, allergenicity, or the like. The variant sequence may be constructed on the basis of the nucleic acid sequence presented as the polypeptide encoding part of SEQ ID NO:1, e.g., a subsequence thereof, and/or by introduction of nucleotide substitutions which do not give rise to another amino acid sequence of the polypeptide encoded by the nucleic acid sequence, but which correspond to the codon usage of the host organism intended for production of the polypeptide, or by introduction of nucleotide substitutions which may give rise to a different amino acid sequence. For a general description of nucleotide substitution see, e.g., Ford et al., 1991, Protein Expression and Purification 2: 95-107. Low-allergenic polypeptides can e.g. be prepared as described above.

The present invention also relates to the non-therapeutic use of a polypeptide encoded by an isolated nucleic acid sequences comprising a nucleic acid sequence encoding a polypeptide and which hybridize under medium stringency conditions, preferably medium-high stringency conditions, more preferably high stringency conditions, and most preferably very high stringency conditions with a nucleic acid probe which hybridizes under the same conditions with the nucleic acid sequence of SEQ ID NO:1 or its complementary strand; or allelic variants and subsequences thereof (Sambrook et al., 1989, supra), as defined herein.

The present invention also relates to the non-therapeutic use of polyeptides encoded by isolated nucleic acid sequences produced by (a) hybridizing a DNA under medium, medium-high, high, or very high stringency conditions with (i) nucleotides nucleotides 124-378 of SEQ ID NO:1, (ii) a subsequence of (i), or (iii) a complementary strand of (i), or (ii); and (b) isolating the nucleic acid sequence. The subsequence is preferably a sequence of at least 100 nucleotides such as a sequence that encodes a polypeptide fragment.

The present invention further relates to methods for producing a mutant nucleic acid sequence, comprising introducing at least one mutation into the mature polypeptide coding sequence of SEQ ID NO:1 or a subsequence thereof, wherein the mutant nucleic acid sequence encodes a polypeptide which consists of amino acids 1-85 of SEQ ID NO:2; or a fragment thereof.

The introduction of a mutation into the nucleic acid sequence to exchange one nucleotide for another nucleotide may be accomplished by site-directed mutagenesis using any of the methods known in the art. Particularly useful is the procedure that utilizes a supercoiled, double stranded DNA vector with an insert of interest and two synthetic primers containing the desired mutation. The oligonucleotide primers, each complementary to opposite strands of the vector, extend during temperature cycling by means of Pfu DNA polymerase. On incorporation of the primers, a mutated plasmid containing staggered nicks is generated. Following temperature cycling, the product is treated with Dpnl which is specific for methylated and hemimethylated DNA to digest the parental DNA template and to select for mutation-containing synthesized DNA. Other procedures known in the art may also be used.

### Nucleic Acid Constructs

The present invention also relates to nucleic acid constructs comprising a nucleic acid sequence operably linked to one or more control sequences that direct the expression of the coding sequence in a suitable expression host. Suitable expression hosts are Bacillus host cells, the DNA of which, when harvested and used as a DNA template in a PCR reaction with SEQ ID NOs:6 and 7 as primers, as described in Example 6, leads to the generation of a PCR fragment of a size of approximately 0.4kb.

Examples of suitable host cells are mentioned in the below section headed "Host cells". Expression will be understood to include any step involved in the production of the polypeptide including, but not limited to, transcription, post-transcriptional modification, translation, post-translational modification, and secretion.

"Nucleic acid construct" is defined herein as a nucleic acid molecule, either single- or double-stranded, which is isolated from a naturally occurring gene or which has been modified to contain segments of nucleic acid combined and juxtaposed in a manner that would not otherwise exist in nature. The term nucleic acid construct is synonymous with the term expression cassette when the nucleic acid construct contains all the control sequences required for expression of a coding sequence for a polypeptide to be used for the non-therapeutic purposes of the present invention. The term "coding sequence" is defined herein as a nucleic acid sequence that directly specifies the amino acid sequence of its protein product. The boundaries of the coding sequence are generally determined by a ribosome binding site (prokaryotes) or by the ATG start codon (eukaryotes) located just upstream of the open reading frame at the 5' end of the mRNA and a transcription terminator sequence located just downstream of the open reading frame at the 3' end of the mRNA. A coding sequence can include, but is not limited to, DNA, cDNA, and recombinant nucleic acid sequences.

An isolated nucleic acid sequence encoding a polypeptide to be used for the non-therapeutic purposes of the present invention may be manipulated in a variety of ways to provide for expression of the polypeptide. Manipulation of the nucleic acid sequence prior to its insertion into a vector may be desirable or necessary depending on the expression vector. The techniques for modifying nucleic acid sequences utilizing recombinant DNA methods are well known in the art.

The term "control sequences" is defined herein to include all components that are necessary or advantageous for the expression of a polypeptide to be used for the non-therapeutic purposes of the present invention. Each control sequence may be native or foreign to the nucleic acid sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide. The term "operably linked" is defined herein as a configuration in which a control sequence is appropriately placed at a position relative to the coding sequence of the DNA sequence such that the control sequence directs the expression of a polypeptide.

The promoter sequence, i.e. a nucleic acid sequence that is recognized by a host cell for expression of the nucleic acid sequence, contains transcriptional control sequences that mediate the expression of the polypeptide. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention in a Bacillus licheniformis host cell which is positive in the test of Example 6 herein are the promoters obtained from Bacillus licheniformis alpha-amylase gene (amyL), Bacillus stearothermophilus maltogenic amylase gene (amyM), Bacillus amyloliquefaciens alpha-amylase gene (amyQ), a CryIIIA promoter (see WO 99/43835), as well as the endogenous L12 promoter of either of the specific Bacillus licheniformis host cells mentioned below.

The transcription terminator sequence, i.e. a sequence recognized by a host cell to terminate transcription, is operably linked to the 3' terminus of the nucleic acid sequence encoding the polypeptide. Any terminator which is functional in the host cell of choice may be used in the present invention.

Preferred terminators for the abovementioned Bacillus licheniformis host cells are the terminators from Bacillus licheniformis alpha-amylase gene (amyL), and the endogenous L12 terminator from either of these host cells.

The control sequence may also be a suitable leader sequence, a nontranslated region of an mRNA which is important for translation by the host cell. The leader sequence is operably linked to the 5' terminus of the nucleic acid sequence encoding the polypeptide. Any leader sequence that is functional in the host cell of choice may be used in the present invention.

The signal peptide coding region encodes an amino acid sequence linked to the amino terminus of a polypeptide which directs the encoded polypeptide into the cell's secretory pathway. In a preferred embodiment, the signal peptide coding region is nucleotides 1-123 of SEQ ID NO:1 which encode amino acids -41 to -1 of SEQ ID NO:2.

According to the SignaIP Version 3.0 software, the predicted signal peptide of SEQ ID NO:2 is amino acids -41 to -2. This means that the predicted mature protein starts at amino acid -1 of SEQ ID NO:2, viz. Ala. However, according to Example 2 herein, the N-terminal of the mature protein starts with amino acid +1 of SEQ ID NO:2, viz. Trp, which means that the signal peptide part spans from amino acids -41 to -1 of SEQ ID NO:2, which is one amino acid longer than predicted.

Therefore amino acids -1 to +85 of SEQ ID NO:2 is an alternative mature form of the L12 protein, the use of which for non-therapeutic purposes in animal feed is also part of the present invention. Accordingly, any claim and any statement herein referring to amino acids 1-85 of SEQ ID NO:2 may therefore also, or alternatively, refer to amino acids -1 to +85 of SEQ ID NO:2. The same is the case for any claim and any statement herein referring to the corresponding part of SEQ ID NO:1: Nucleotides 121-378 of SEQ ID NO:1 may be referred to in addition to, or in the alternative to, nucleotides 124-378 of SEQ ID NO:1.

Likewise, any reference herein to the signal part of SEQ ID NO:2 may also, or in the alternative, be to amino acids -41 to -2 of SEQ ID NO:2. The corresponding part of SEQ ID NO:1 is nucleotides 1-120 of SEQ ID NO:1. Any claim and any statement herein referring to the signal peptide part may therefore also, or alternatively, refer to nucleotides 1-120 of SEQ ID NO:1.

The SignaIP method V. 3.0 is described in Bendtsen et al in Journal of Molecular Biology 2004, 340(4), pp. 783-95. See also Nielsen et al in Proceedings of the Sixth International Conference on Intelligent Systems for Molecular Biology (ISMB 6), AAAI Press, Menlo Park, California, pp 122-130, 1998 (V. 2.0); and Nielsen et al in Protein Engineering 10, 1-6 (1997) (V. 1.1).

### Expression Vectors

The present invention also relates to recombinant expression vectors comprising a nucleic acid construct, such as recombinant expression vectors comprising a nucleic acid sequence encoding a polypeptide to be used according to the present invention, a promoter, and transcriptional and translational stop signals. The various nucleic acid and control sequences described above may be joined together to produce a recombinant expression vector which may include one or more convenient restriction sites to allow for insertion or substitution of the nucleic acid sequence encoding the polypeptide at such sites. Alternatively, the nucleic acid sequence may be expressed by inserting the nucleic acid sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (e.g., a plasmid or virus) which can be conveniently subjected to recombinant DNA procedures and can bring about the expression of the nucleic acid sequence. The choice of the vector will typically depend on the compatibility of the vector with the host cell into which the vector is to be introduced. The vectors may be linear or closed circular plasmids.

The vector may be an autonomously replicating vector, i.e., a vector which exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the dal genes from Bacillus subtilis or Bacillus licheniformis, or markers which confer antibiotic resistance such as ampicillin, kanamycin, chloramphenicol or tetracycline resistance.

The vectors of the present invention preferably contain an element(s) that permits stable integration of the vector into the host cell's genome or autonomous replication of the vector in the cell independent of the genome.

For integration into the host cell genome, the vector may rely on the nucleic acid sequence encoding the polypeptide or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleic acid sequences. On the other hand, the vector may be integrated into the genome of the host cell by non-homologous recombination.

For autonomous replication, the vector may further comprise an origin of replication enabling the vector to replicate autonomously in the host cell in question. Examples of bacterial origins of replication are the origins of replication of plasmids pBR322, pUC19, pACYC177, and pACYC184 permitting replication in E. coli, and pUB110, pE194, pTA1060, and pAMß1 permitting replication in Bacillus. The origin of replication may be one having a mutation which makes its functioning temperature-sensitive in the host cell (see, e.g., Ehrlich, 1978, Proceedings of the National Academy of Sciences USA 75: 1433).

More than one copy of a nucleic acid sequence may be inserted into the host cell to increase production of the gene product. An increase in the copy number of the nucleic acid sequence can be obtained by integrating at least one additional copy of the sequence into the host cell genome or by including an amplifiable selectable marker gene with the nucleic acid sequence where cells containing amplified copies of the selectable marker gene, and thereby additional copies of the nucleic acid sequence, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

The polypeptide may also be co-expressed together with at least one other component of interest for animal feed, such as a polypeptide with a desired enzymatic activity for use in animal feed. The polypeptide and the at least one other polypeptide may be co-expressed from different vectors, from one vector, or using a mixture of both techniques.

### Host Cells

The invention furthermore relates to the use for non-therapeutic purposes in animal feed of a recombinant Bacillus host cell comprising a nucleic acid construct of the invention and/or an expression vector of the invention. The DNA of the host cell, when harvested and used as a DNA template in a PCR reaction with SEQ ID NOs:6 and 7 as primers, leads to the generation of a PCR fragment of a size of approximately 0.4kb.

In a particular embodiment, the PCR fragment, when purified and sequenced encodes an amino acid sequence which has at least 33% identity to amino acids 1-85 of SEQ ID NO:2.

The particular embodiments (in particular those disclosed in the sections headed "The Polypeptide, its Characteristics, and Use" and "Identity and Hybridization, Fragments and Variants") are also applicable to the host cells of the invention. E.g., in further particular embodiments of the Bacillus host cell of the invention, the PCR fragment, when purified and sequenced, encodes an amino acid sequence which has a degree of identity to amino acids 1-85 of SEQ ID NO:2 of at least 35%, or a least 37%, 40%, 42%, 45%, 47%, 50%, 52%, 55%, 57%, 60%, 62%, 65%, 67%, 70%, 72%, 75%, 77%, 80%, 82%, 85%, 87%, 90%, 92%, 95%, 97%, or at least 99%.

In still further particular embodiments, the Bacillus host cell is a Bacillus licheniformis host cell, preferably selected from the following strains of Bacillus licheniformis: ATCC 14580 (=NCIB 9375), NCIMB 6346 (=DSM 8785), NCTC 1024, NCTC 1025, NCTC 2120, NCTC 7589, NCTC 9932, ATCC 21424, NCIMB 10689, and ATCC 53757. A preferred subgroup includes Bacillus licheniformis ATCC 14580 (=NCIB 9375), and Bacillus licheniformis NCIMB 6346 (=DSM 8785).

These host cells are advantageously used in the recombinant production of the polypeptides. A vector comprising a nucleic acid sequence encoding a polypeptide to be used according to the present invention is introduced into the host cell so that the vector is maintained as a chromosomal integrant or as a self-replicating extra-chromosomal vector as described earlier. The term "host cell" encompasses any progeny of the parent cell that is not identical to the parent cell due to mutations that occur during replication.

The introduction of a vector into a bacterial host cell may, for instance, be effected by protoplast transformation (see, e.g., Chang and Cohen, 1979, Molecular General Genetics 168: 111-115), using competent cells (see, e.g., Young and Spizizin, 1961, Journal of Bacteriology 81: 823-829, or Dubnau and Davidoff-Abelson, 1971, Journal of Molecular Biology 56: 209-221), electroporation (see, e.g., Shigekawa and Dower, 1988, Biotechniques 6: 742-751), or conjugation (see, e.g., Koehler and Thorne, 1987, Journal of Bacteriology 169: 5771-5278).

### Methods of Production

The invention also relates to a methods for producing a polypeptide to be used according to the invention, the method comprising (a) cultivating a recombinant host cell of the invention to produce a supernatant comprising the polypeptide; and (b) recovering the polypeptide.

The invention furthermore relates to a method for producing such a polypeptide comprising (a) cultivating a strain of Bacillus, the DNA of which, when harvested and used as a DNA template in a PCR reaction with SEQ ID NOs:6 and 7 as primers, leads to the generation of a PCR fragment of a size of approximately 0.4kb; and (b) recovering the polypeptide.

Examples of host cells are mentioned in the above section headed "Host cells".

The present invention also relates to methods for producing a polypeptide to be used in accordance to the present invention comprising (a) cultivating a recombinant Bacillus licheniformis ATCC 14580 (=NCIB 9375) or Bacillus licheniformis NCIMB 6346 (=DSM 8785) host cell under conditions conducive for production of the polypeptide, wherein the host cell comprises a mutant nucleic acid sequence comprising at least one mutation in nucleotides 124-378 of SEQ ID NO:1, in which the mutant nucleic acid sequence encodes a polypeptide which (i) consists of amino acids 1-85 of SEQ ID NO:2, or (ii) is a variant of the sequence of (i), wherein the variant comprises a substitution, deletion, and/or insertion of one or more amino acids, or (iii) is an allelic variant of the sequence of (i), or (iv) is a fragment of the sequence of (i).

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, repeated fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.

The polypeptides may be detected using methods known in the art that are specific for the polypeptides. These detection methods may include use of specific antibodies; SDS-PAGE gel analysis revealing a band of a relative molecular weight, Mr, of about 12kDa; and/or determination of the N-terminal sequence, on purified samples and/or on the band of a Mr of 12 kDa, as cut-out from the SDS-PAGE gel. In the latter case, the N-terminal should preferably have an identity to SEQ ID NO:5 of at least 33%, or at least 35%, 37%, 40%, 42%, 45%, 47%, 50%, 52%, 55%, 57%, 60%, 62%, 65%, 67%, 70%, 72%, 75%, 77%, 80%, 82%, 85%, 87%, 90%, 92%, 95%, 97%, or at least 99%, the percentage identity being determined as generally described above).

The resulting polypeptide may be recovered by methods known in the art. For example, the polypeptide may be recovered from the nutrient medium by conventional procedures including, but not limited to, centrifugation, filtration (such as ultrafiltration and/or diafiltration), extraction, spray-drying, evaporation, or precipitation.

The polypeptides may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989).

### Compositions and Uses

In a still further aspect, the present invention relates to compositions comprising a polypeptide and/or a Bacillus strain of the present invention.

The polypeptide compositions may be prepared in accordance with methods known in the art and may be in the form of a liquid or a dry composition. For instance, the polypeptide composition may be in the form of a granulate or a microgranulate. The polypeptide to be included in the composition may be stabilized in accordance with methods known in the art.

Particular examples of compositions of the invention are the following:
An animal feed additive comprising (a) i) a polypeptide having amino acids 1-126 of SEQ ID NO:4 and/or ii) a polypeptide comprising an amino acid sequence which has a degree of identity to amino acids 1-85 of SEQ ID NO:2 of at least 33%, as determined by the Needle program, using the BLOSUM62 substitution matrix, a gap opening penalty of 10, and a gap extension penalty of 0.5;; and (b) at least one fat-soluble vitamin, (c) at least one water-soluble vitamin, (d) at least one trace mineral, and/or (e) at least one macro mineral;
an animal feed composition having a crude protein content of 50 to 800 g/kg and comprising i) a polypeptide having amino acids 1-126 of SEQ ID NO:4 and/or ii) a polypeptide comprising an amino acid sequence which has a degree of identity to amino acids 1-85 of SEQ ID NO:2 of at least 33%, as determined by the Needle program, using the BLOSUM62 substitution matrix, a gap opening penalty of 10, and a gap extension penalty of 0.5;;
an animal feed additive comprising (a) a strain of Bacillus as defined in the section headed "Bacterial strains; Probiotic Bacillus strains"; and (b) at least one fat-soluble vitamin, (c) at least one water-soluble vitamin, (d) at least one trace mineral, and/or (e) at least one macro mineral; and
an animal feed composition having a crude protein content of 50 to 800 g/kg and comprising a strain of Bacillus as defined in the section headed "Bacterial strains; Probiotic Bacillus strains".

The so-called premixes are examples of animal feed additives of the invention. A premix designates a preferably uniform mixture of one or more micro-ingredients with diluent and/or carrier. Premixes are used to facilitate uniform dispersion of micro-ingredients in a larger mix.

Examples of preferred uses according to the invention are given above (in the sections headed "The Polypeptide, its Characteristics, and Use" and "Bacterial strains; Probiotic Bacillus strains"), and further detailed below.

### Animal Feed

The term animal includes all animals. Examples of animals are non-ruminants, and ruminants. Ruminant animals include, for example, animals such as sheep, goat, and cattle, e.g. cow such as beef cattle and dairy cows. In a particular embodiment, the animal is a non-ruminant animal. Non-ruminant animals include mono-gastric animals; e.g. pig or swine (including, but not limited to, piglets, growing pigs, and sows); poultry such as turkeys, ducks and chickens (including but not limited to broiler chicks, layers); fish (including but not limited to salmon, trout, tilapia, catfish and carp); and crustaceans (including but not limited to shrimp and prawn).

The term feed or feed composition means any compound, preparation, mixture, or composition suitable for, or intended for intake by an animal.

In the use according to the invention the polypeptide and/or the Bacillus strain can be fed to the animal before, after, or simultaneously with the diet. The latter is preferred.

In a particular embodiment, the polypeptide, in the form in which it is added to the feed, or when being included in a feed additive, is well-defined. The term well-defined means that the polypeptide preparation is at least 50% pure, determined as described previously, or by Size-exclusion chromatography (see Example 12 of WO 01/58275). In other particular embodiments the well-defined polypeptide preparation is at least 60, 70, 80, 85, 88, 90, 92, 94, or at least 95% pure.

A well-defined polypeptide preparation is advantageous. For instance, it is much easier to dose correctly to the feed a polypeptide that is essentially free from interfering or contaminating other polypeptides. The term dose correctly refers in particular to the objective of obtaining consistent and constant results, and the capability of optimising dosage based upon the desired effect.

For the use in animal feed, however, the polypeptide need not be that pure; it may e.g. include other polypeptides such as animal feed enzymes, in which case it could be termed a polypeptide preparation.

The polypeptide preparation can be (a) added directly to the feed (or used directly in a treatment process of proteins), or (b) it can be used in the production of one or more intermediate compositions such as feed additives or premixes that is subsequently added to the feed (or used in a treatment process). The degree of purity described above refers to the purity of the original polypeptide preparation, whether used according to (a) or (b) above.

Polypeptide preparations with purities of this order of magnitude are in particular obtainable using recombinant methods of production, whereas they are not so easily obtained and also subject to a much higher batch-to-batch variation when the polypeptide is produced by traditional fermentation methods.

In the present context, the term Feed Conversion Ratio, or FCR, is used synonymously with the term feed conversion. The FCR is calculated as the feed intake in g/animal relative to the weight gain in g/animal, see e.g. Table 2 in Example 5.

The polypeptide can be added to the feed in any form, be it as a relatively pure polypeptide, or in admixture with other components intended for addition to animal feed, i.e. in the form of animal feed additives, such as the so-called pre-mixes for animal feed.

Apart from the polypeptide and/or the Bacillus strain, the animal feed additives of the invention contain at least one fat-soluble vitamin, and/or at least one water soluble vitamin, and/or at least one trace mineral, and/or at least one macro mineral.

Further, optional, feed-additive ingredients are colouring agents, e.g. carotenoids such as beta-carotene, astaxanthin, and lutein; aroma compounds; stabilisers; antimicrobial peptides; polyunsaturated fatty acids; reactive oxygen generating species; and/or at least one enzyme selected from amongst phytase (EC 3.1.3.8 or 3.1.3.26); xylanase (EC 3.2.1.8); galactanase (EC 3.2.1.89); alpha-galactosidase (EC 3.2.1.22); protease (EC 3.4.- .- ), phospholipase A1 (EC 3.1.1.32); phospholipase A2 (EC 3.1.1.4); lysophospholipase (EC 3.1.1.5); phospholipase C (EC 3.1.4.3); phospholipase D (EC 3.1.4.4); amylase such as, for example, alpha-amylase (EC 3.2.1.1); and/or beta-glucanase (EC 3.2.1.4 or EC 3.2.1.6). In a particular embodiment these other enzymes are well-defined (as defined above for polypeptide preparations).

Examples of antimicrobial peptides (AMP's) are CAP18, Leucocin A, Tritrpticin, Protegrin-1, Thanatin, Defensin, Lactoferrin, Lactoferricin, and Ovispirin such as Novispirin (Robert Lehrer, 2000), Plectasins, and Statins, including the compounds and polypeptides disclosed in WO 03/044049 and WO 03/048148, as well as variants or fragments of the above that retain antimicrobial activity.

Examples of antifungal polypeptides (AFP's) are the Aspergillus giganteus, and Aspergillus niger peptides, as well as variants and fragments thereof which retain antifungal activity, as disclosed in WO 94/01459 and WO 02/090384.

Examples of polyunsaturated fatty acids are C18, C20 and C22 polyunsaturated fatty acids, such as arachidonic acid, docosohexaenoic acid, eicosapentaenoic acid and gamma-linoleic acid.

Examples of reactive oxygen generating species are chemicals such as perborate, persulphate, or percarbonate; and enzymes such as an oxidase, an oxygenase or a syntethase.

Usally fat- and water-soluble vitamins, as well as trace minerals form part of a so-called premix intended for addition to the feed, whereas macro minerals are usually separately added to the feed. Either of these composition types, when enriched with a polypeptide or a Bacillus strain of the invention, is an animal feed additive of the invention.

In a particular embodiment, the animal feed additive of the invention is intended for being included (or prescribed as having to be included) in animal diets or feed at levels of 0.01 to 10.0%; more particularly 0.05 to 5.0%; or 0.2 to 1.0% (% meaning g additive per 100 g feed). This is so in particular for premixes.

The following are non-exclusive lists of examples of these components:

Examples of fat-soluble vitamins are vitamin A, vitamin D3, vitamin E, and vitamin K, e.g. vitamin K3.

Examples of water-soluble vitamins are vitamin B12, biotin and choline, vitamin B1, vitamin B2, vitamin B6, niacin, folic acid and panthothenate, e.g. Ca-D-panthothenate.

Examples of trace minerals are manganese, zinc, iron, copper, iodine, selenium, and cobalt.

Examples of macro minerals are calcium, phosphorus and sodium.

The nutritional requirements of these components (exemplified with poultry and piglets/pigs) are listed in Table A of WO 01/58275. Nutritional requirement means that these components should be provided in the diet in the concentrations indicated.

In the alternative, the animal feed additive of the invention comprises at least one of the individual components specified in Table A of WO 01/58275. At least one means either of, one or more of, one, or two, or three, or four and so forth up to all thirteen, or up to all fifteen individual components. More specifically, this at least one individual component is included in the additive of the invention in such an amount as to provide an in-feed-concentration within the range indicated in column four, or column five, or column six of Table A.

Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore such fish diets usually have a crude fat content of 200-310 g/kg.

An animal feed composition according to the invention has a crude protein content of 50-800 g/kg, and furthermore comprises at least one polypeptide and/or at least one Bacillus strain as described and/or claimed herein.

Furthermore, or in the alternative (to the crude protein content indicated above), the animal feed composition of the invention has a content of metabolisable energy of 10-30 MJ/kg; and/or a content of calcium of 0.1-200 g/kg; and/or a content of available phosphorus of 0.1-200 g/kg; and/or a content of methionine of 0.1-100 g/kg; and/or a content of methionine plus cysteine of 0.1-150 g/kg; and/or a content of lysine of 0.5-50 g/kg.

In particular embodiments, the content of metabolisable energy, crude protein, calcium, phosphorus, methionine, methionine plus cysteine, and/or lysine is within any one of ranges 2, 3, 4 or 5 in Table B of WO 01/58275 (R. 2-5).

Crude protein is calculated as nitrogen (N) multiplied by a factor 6.25, i.e. Crude protein (g/kg)= N (g/kg) x 6.25. The nitrogen content is determined by the Kjeldahl method (A.O.A.C., 1984, Official Methods of Analysis 14th ed., Association of Official Analytical Chemists, Washington DC).

Metabolisable energy can be calculated on the basis of the NRC publication Nutrient requirements in swine, ninth revised edition 1988, subcommittee on swine nutrition, committee on animal nutrition, board of agriculture, national research council. National Academy Press, Washington, D.C., pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.

The dietary content of calcium, available phosphorus and amino acids in complete animal diets is calculated on the basis of feed tables such as Veevoedertabel 1997, gegevens over chemische samenstelling, verteerbaarheid en voederwaarde van voedermiddelen, Central Veevoederbureau, Runderweg 6, 8219 pk Lelystad. ISBN 90-72839-13-7.

In a particular embodiment, the animal feed composition of the invention contains at least one vegetable protein or protein source. It may also contain animal protein, such as Meat and Bone Meal, and/or Fish Meal, typically in an amount of 0-25%. The term vegetable proteins as used herein refers to any compound, composition, preparation or mixture that includes at least one protein derived from or originating from a vegetable, including modified proteins and protein-derivatives. In particular embodiments, the protein content of the vegetable proteins is at least 10, 20, 30, 40, 50, or 60% (w/w).

Vegetable proteins may be derived from vegetable protein sources, such as legumes and cereals, for example materials from plants of the families Fabaceae (Leguminosae), Cruciferaceae, Chenopodiaceae, and Poaceae, such as soy bean meal, lupin meal and rapeseed meal.

In a particular embodiment, the vegetable protein source is material from one or more plants of the family Fabaceae, e.g. soybean, lupine, pea, or bean.

In another particular embodiment, the vegetable protein source is material from one or more plants of the family Chenopodiaceae, e.g. beet, sugar beet, spinach or quinoa.

Other examples of vegetable protein sources are rapeseed, sunflower seed, cotton seed, and cabbage.

Other examples of vegetable protein sources are cereals such as barley, wheat, rye, oat, maize (corn), rice, triticale, and sorghum.

In still further particular embodiments, the animal feed composition of the invention contains 0-80% maize; and/or 0-80% sorghum; and/or 0-70% wheat; and/or 0-70% Barley; and/or 0-30% oats; and/or 0-30% rye; and/or 0-40% soybean meal; and/or 0-25% fish meal; and/or 0-25% meat and bone meal; and/or 0-20% whey.

Animal diets can e.g. be manufactured as mash feed (non pelleted) or pelleted feed. Typically, the milled feed-stuffs are mixed and sufficient amounts of essential vitamins and minerals are added according to the specifications for the species in question. The polypeptide(s) and/or the Bacillus strain can be added as solid or liquid formulations. For example, a solid polypeptide formulation is typically added before or during the mixing step; and a liquid polypeptide preparation is typically added after the pelleting step. The polypeptide may also be incorporated in a feed additive or premix.

The final polypeptide concentration in the diet is within the range of 0.01-200 mg protein per kg diet, for example in the range of 0.1-20 mg protein per kg animal diet.

The polypeptide and/or the Bacillus strain should of course be applied in an effective amount, i.e. in an amount adequate for improving feed conversion.

It is at present contemplated that the polypeptide is administered in one or more of the following amounts (dosage ranges): 0.01-200; 0.01-100; 0.5-100; 1-50; 5-100; 10-100; 0.05-50; 1-10; or 0.10-10, all these ranges being in mg polypeptide protein per kg feed (ppm). In a particular embodiment, the dosage range is 1-9, 1-8, 2-7, 2-6, or 2-5 ppm. Examples of particularly preferred dosage range are; 0.5-15.0, 1.0-12.5, 1.5-10.0, and 2.5-7.5 ppm. The amount of the polypeptide is determined as described for the L12 protein in Example 10.

It is at present contemplated that the Bacillus strain is administered in one or more of the following amounts (dosage ranges): 10 E2-14, 10 E4-12, 10 E6-10, 10 E7-9, preferably 10 E8 CFU/g of feed (the designation E meaning exponent, viz., e.g., 10 E2-14 means 10²-10¹⁴).

For determining mg polypeptide protein per kg feed, the polypeptide is purified from the feed composition, and the dosage in mg polypeptide protein per kg feed is calculated, for example as described in Example 10. The same principles apply for determining mg polypeptide protein in feed additives.

### Deposit of Biological Material

The following biological material, which was isolated from intestinal contents of broilers, as described in Example 8, has been deposited under the terms of the Budapest Treaty with DSMZ (DSMZ-Deutsche Sammlung von Mikroorganismen und Zellkulturen GmbH, Mascheroder Weg 1b, D-38124 Braunschweig, Germany) and given the following accession numbers:

| Deposit | Accession Number | Date of Deposit |
|---|---|---|
| Enterococcus faecalis | DSM 18047 | 10 March |
| 2006 | | |
| Lactobacillus salivarius | DSM 18070 | 16 March 2006 |

The deposits were made by Novozymes A/S, Krogshoejvej 36, DK-2880, Denmark.

### Examples

### Example 1: Shake flask fermentation of Bacillus licheniformis

Bacillus licheniformis ATCC 14580 was propagated overnight at 37°C on TY agar medium (TY broth solidified with 2% agar) and inoculated into a shake flask containing 100 ml TY broth with the following composition: Tryptone: 20 g/I, Yeast extract: 5 g/l, FeCl₂, 4H₂O: 7 mg/l, MnCl₂, 4H₂O: 1 mg/l, MgSO₄, 7H₂O: 15 mg/l, pH 7.3. The shake flask was incubated at 37°C for 20 hours with a shaking speed of 225 rpm. The cells were removed by centrifugation.

SDS polyacrylamide gel electrophoresis of the supernatant revealed a band of a relative molecular weight of 12 kDa corresponding to the desired polypeptide. The polypeptide is designated "the L12 protein," due to its relative molecular weight of 12 kDa, by SDS-PAGE (however, the theoretical molecular weight can be calculated to 9591.56 Da).

### Example 2: Purification of Bacillus licheniformis shake flask fermentation

The fermentation broth from Example 1 was centrifuged (20000 x g, 20 min) and the supernatants were carefully decanted from the precipitates. The combined supernatants were filtered through a Seitz EKS plate in order to remove the Bacillus cells. The EKS filtrate was ultrafiltrated and diafiltrated on Filtron 3k cut-off cassettes in order to concentrate the proteins and reduce the conductivity in the Seitz EKS filtrate. The Filtron concentrate was filtered through another Seitz EKS plate.

The pH of the concentrate was adjusted to pH 4.5 with 20% CH₃COOH. Something (not the L12 protein) in the solution precipitated by the pH adjustment and this precipitate was removed by filtering through a Seitz K-250 plate. The K-250 filtrate was applied to a 100ml SP-sepharose FF column equilibrated in 20mM CH₃COOH, 50mM H₃BO₃, 1 mM CaCl₂, adjusted to pH 4.5 with NaOH. After washing the column extensively with the equilibration buffer, the L12 protein was eluted with a linear NaCl gradient (0 --> 0.5M) in the same buffer. L12 protein containing fractions were identified by SDS-PAGE analysis and these fractions were pooled and diluted 10 times with demineralized water to reduce the conductivity of the pool. The pool was applied to an 8ml SOURCE S column equilibrated in the same equilibration buffer (20mM CH₃COOH, 50mM H₃BO₃, 1mM CaCl₂, adjusted to pH 4.5 with NaOH) and after washing the column extensively with the equilibration buffer, the L12 protein was eluted with a linear NaCl gradient (0 --> 1.0M) in the same buffer.

Fractions from the column were analysed by SDS-PAGE analysis and L12 protein containing fractions were pooled, the pH was adjusted to pH 8 with 3% NaOH and the pool was left in the cold room to the next day. The pH 8 adjustment caused the L12 protein to precipitate, and the next day the L12 precipitate was collected by centrifugation (5000 x g, 10 min).

The L12 protein precipitate was washed with 20mM Tris/HCl, pH 8 to increase the purity of the precipitated L12 protein. After a second centrifugation (5000 x g, 10 min) the L12 protein precipitate was dissolved in a minimal volume of (20mM CH₃COOH, 50mM H₃BO₃, 1mM CaCl₂, 100mM NaCl adjusted to pH 4.5 with NaOH) and applied to a 300ml Superdex 75 size-exclusion column equilibrated in the same buffer.

The Superdex 75 column was eluted with the same buffer and fractions from the column were analysed by SDS-PAGE analysis. Fractions, where only one band was seen on the coomassie stained SDS-PAGE gel, were pooled as the purified L12 protein preparation. Glycerol was added to the pooled L12 fractions to 50% (w/w) final concentration.

The glycerol formulated L12 protein was stored cold in a refrigerator. The preparation was essentially pure as determined on a coomassie stained SDS-PAGE gel (one band).

### Characteristics:

The relative molecular weight as determined by SDS-PAGE was: Mr = 12kDa.
The N-terminal sequence was: WVNPGYHYQYPSEGG (SEQ ID NO:5)

### Example 3: Fed-batch fermentation of Bacillus licheniformis

A fed-batch fermentation process of Bacillus licheniformis ATCC 14580 was conducted as described below. All media were sterilized by methods known in the art. Unless otherwise described, tap water was used. The ingredient concentrations referred to in the below recipes are before any inoculation.

### Media

LB agar: 10 g/I peptone from casein (such as, Fluka catalogue no. 95039, tryptic digest from casein); 5 g/I yeast extract (manufactured by autolysis of Saccharomyces cerevisiae, e.g. catalogue no. 9512 from Organotechnie S.A., 27, avenue Jean Mermoz, F-93120 La Courneuve, France); 10 g/I sodium chloride; 12 g/l Bacto-agar (LB-agar (Miller), Merck catalogue no. 110283) adjusted to pH 7.0 +/- 0.2.

M-9 buffer: Di-Sodiumhydrogenphosphate, 2H₂O 8.8 g/I; potassiumdihydrogenphosphate 3 g/l; sodium chloride 4 g/l; magnesium sulphate, 7H₂O 0.2 g/I (deionized water is used in this buffer).

PRK-50: 110 g/I soy grits; Di-sodiumhydrogenphosphate, 2H₂O 5 g/I; antifoam (such as, e.g., Struktol SB2121, Schill & Seilacher, Hamburg, Germany) 1 ml/l; pH adjusted to 8.0 with NaOH/H₃PO₄ before sterilization.

Make-up medium: Tryptone (Casein hydrolysate such as, e.g., Bacto^{™} Tryptone pancreatic digest of casein catalogue no. 211699) 30 g/I; magnesium sulphate, 7H₂O 4 g/I; di-potassiumhydrogenphosphate 7 g/l; di-sodiumhydrogenphosphate, 2H₂O 7 g/l; di-ammoniumsulphate 4 g/I; citric acid 0.78 g/l; vitamins (thiamin-dichlorid 34.2 mg/l; riboflavin 2.9 mg/l; nicotinic acid 23 mg/l; calcium D-pantothenate 28.5 mg/l; pyridoxal-HCl 5.7 mg/l; D-biotin 1.1 mg/l; folic acid 2.9 mg/l); trace metals (MnSO₄, H₂O 39.2 mg/l; FeSO₄, 7H₂O 157 mg/l; CuSO₄, 5H₂O 15.6 mg/l; ZnCl₂ 15.6 mg/l); Antifoam (Struktol SB2121, see above) 1.25 ml/l; pH adjusted to 6.0 with NaOH/H₃PO₄ before sterilization. Monopropylene glycol (MPG) 24 ml/l was added 28 and 47 hours after inoculation (i.e. after approximately 1 and 2 days, respectively), in total 48 ml/I of MPG was added.
Feed-medium: Glucose,1H₂O 820 g/l

### Fermentation Procedure:

Bacillus licheniformis ATCC 14580 was grown on LB agar slants for one day at 37°C. The agar was then washed with M-9 buffer, and the optical density (OD) at 650 nm of the resulting cell suspension was measured. Inoculum shake flasks (with 100 ml medium PRK-50) were inoculated with an inoculum of OD (650 nm) x ml cell suspension = 0.1 (which means that the required amount of inoculum in ml is found by dividing 0.1 by the OD (650nm) of the inoculum cell suspension). The shake flasks were incubated at 37°C at 300 rpm for 20 hr.

The fermentors used were standard lab fermentors equipped with a temperature control system, pH control with ammonia water and phosphoric acid, dissolved oxygen electrode to measure >20% oxygen saturation through the entire fermentation.

The fermentation in the main fermentor (fermentation tank) was started by inoculating the main fermentor with the growing culture from an inoculum shake flask. The inoculated volume was 10% of the make-up medium (80 ml for 720 ml make-up medium, resulting in 800 ml initial broth after inoculation).

The fermentation parameters were: Temperature 41 °C; pH between 6.8 and 7.2 (using ammonia water and phosphoric acid, control 6.8 (ammonia water), 7.2 phosphoric acid). Aeration: 1.5 liter/min/kg of the fermentation broth weight, agitation: 1500 rpm.

Feed-medium was added as follows: Initial feed rate 0.05 g/min/kg at the start of the fermentation, increasing linearly to 0.16 g/ming/kg after 8 hours, and remaining at 0.16 g/min/kg until the end of the fermentation (by reference to the starting weight of the fermentation broth, just after the inoculation).

After 3 days (70 hours) the fermentation broth was harvested and purified as described in Example 4 below.

### Example 4: Purification of fed-batch fermentation of Bacillus licheniformis

The fermentation broth from Example 3 was centrifuged (20000 x g, 20 min) and the supernatants were carefully decanted from the precipitates. The combined supernatants were filtered through a Seitz K-250 plate and then through a Seitz EKS plate in order to remove the rest of the Bacillus host cells. The conductivity of the EKS filtrate was 10 mS/cm. 100ml EKS filtrate was diluted 10x in 20mM CH₃COOH, 50mM H₃BO₃, 1mM CaCl₂, adjusted to pH 4.5 with NaOH and pH of the diluted EKS filtrate was adjusted to pH 4.5 with 20% CH₃COOH. The diluted EKS filtrate was applied to a 19ml SP-sepharose FF column equilibrated in 20mM CH₃COOH, 50mM H₃BO₃, 1mM CaCl₂, adjusted to pH 4.5 with NaOH. After washing the column extensively with the equilibration buffer, the L12 protein was eluted with a linear NaCl gradient (0 --> 0.5M) in the same buffer. Fractions containing the L12 protein were identified by SDS-PAGE analysis and pooled and diluted 10 times with demineralized water to reduce the conductivity of the pool. The pool was applied to an 8ml SOURCE S column equilibrated in the same equilibration buffer (20mM CH₃COOH, 50mM H₃BO₃, 1mM CaCl₂, adjusted to pH 4.5 with NaOH) and after washing the column extensively with the equilibration buffer, the L12 protein was eluted with a linear NaCl gradient (0 --> 1.0M) in the same buffer. Fractions from the column were analysed by SDS-PAGE analysis and fractions containing the L12 protein were pooled and applied to a 120ml Superdex 75 size-exclusion column equilibrated in 20mM CH₃COOH, 50mM H₃BO₃, 100mM NaCl, 1mM CaCl₂, adjusted to pH 4.5 with NaOH. The Superdex 75 column was eluted with the same buffer and fractions from the column were analysed by SDS-PAGE analysis. Fractions giving rise to a strong band at 12 kDa on the coomassie stained SDS-PAGE gel were pooled as the purified L12 protein preparation. The preparation was at least 90% pure judged from a coomassie stained SDS-PAGE gel, and the relative molecular weight as determined by SDS-PAGE was Mr = 12kDa. The N-terminal sequence was: WNVPGYHYQY (amino acids 1-10 of SEQ ID NO:5).

### Example 5: Performance in animal feed in vivo (cage trial)

The performance of the L12 protein in animal feed was evaluated in a broiler chicken growth trial with the following parameters:

| | |
|---|---|
| Growth trial: | Day 8 to day 29 (day 0 = day of hatch) |
| Treatments: | A: Control; B: 5 mg purified L12 protein per kg feed |
| Diets: | Maize / SBM48 diet (see feed composition, Table 1) |

After mixing the feed was pelleted at about 70° C (3 x 25 mm). The L12 protein was diluted in 500 ml of water and sprayed onto the pellets. For the control treatment the same quantity of water was sprayed onto the pellets.

| | |
|---|---|
| Replicates: | 8 groups of 6 male chicken, ROSS PM3, per treatment |
| Feeding: | Pellets ad libitum |

**Table 1: Feed composition of the experimental diet**

| Ingredients (%): | |
|---|---|
| Maize ⁴ | 64.34 |
| SBM 48 ⁵ | 29.20 |
| Soybean oil | 2.50 |
| DL-Methionine | 0.08 |
| Mono Calcium Phosphate | 1.50 |
| Limestone | 1.18 |
| Salt | 0.10 |
| TiO₂ ¹ | 0.10 |
| Premix no. UH 12699002 ² | 1.00 |

| Calculated content: | |
|---|---|
| Crude protein (%) | 19.0 |
| ME_{N} (MJ/kg) ³ | 12.8 |
| Crude fat (%) | 6.2 |
| Lysine (%) | 1.02 |
| Methionine (%) | 0.39 |
| Methionine + Cystine (%) | 0.71 |

| | |
|---|---|
| ¹ TiO₂ as indigestible marker was included in the feed mixture ² Commercially available from DSM Nutritional Products NV, Dorpsstraat 4, B-9800 Deinze, Belgium, and including Lasalocid Sodium ³ Calculated with EC-equation (EEC (1986) Directive de la Commission du 9 avril 1986 fixant la méthode de calcul de la valeur énérgétique des aliments composés destinés à la volaille; Journal Officiel des Communautés Européennes, L 130, 53-54) ⁴ Raw material for feeding stuff; available from Moulin Moderne Hirsingue, Hirsingue, France ⁵ Soybean meal, residues of oil manufacture, raw material for feeding stuff; available from Moulin Moderne Hirsinque, Hirsinque, France | |

**Table 2: Performance of male broiler chicken; mean ± stdev**

| **Product** | **Control** | **L12** |
|---|---|---|
| Treatment | A | B |
| Dose (protein/kg) | 0 | 5 mg |
| Cages x birds | 8 x 6 | 8 x 6 |
| Weight gain (g/bird) | 1308 ^{A} | 1375 ^{A} |
| day 8-29 | ± 61 | ± 80 |
| % | *100.0* | *105.2* |
| Feed intake (g/bird) | 2095 ^{A} | 2119 ^{A} |
| day 8-29 | ± 103 | ± 90 |
| % | *100.0* | *101.1* |
| Feed conversion (g feed/g gain) | 1.603 ^{A} | 1.542 ^{B} |
| day 8-29 | ± 0.028 | ± 0.038 |
| % | *100.0* | *96.2* |

| | | |
|---|---|---|
| Newman-Keuls test: Means within a row, not sharing a common superscript, are significantly different (p < 0.05) | | |

### Example 6: Bacillus strains with L12-like genes, as identified by PCR

Genes similar to the gene encoding the L12 protein (SEQ ID NO:1) were identified in a number of other Bacillus licheniformis strains by PCR. DNA for use as a template for the PCR reaction was isolated from eleven different Bacillus licheniformis strains grown overnight at 37°C on TY agar plates (for recipe, see Example 1). One inoculation tube with cells from each strain were suspended in 0.1 ml H₂O and boiled for 10 min, centrifuged, and 5 microliter supernatant from each was used as DNA template in PCR reactions as described below.

The PCR reactions were run in "Pure TaqTM Ready-To_GoTM PCR Beads" from Amersham Biosciences: 5 microliter DNA template + 2 x 1 microliter of primer Pep481 (SEQ ID NO:6) and Pep482 (SEQ ID NO:7) + 18 microliter H₂O.

PCR program: 1) 95°C 3min; 2) 95°C 10sec; 3) 65°C 30sec -1°C pr. cycle; 4) 72°C 1min; 5) Go To 2) 9 times; 6) 95°C 10sec; 7) 55°C 30sec; 8) 72°C 1min; 9) Go To 6) 19 times; 10) 72°C 5min; 11) 4°C forever, which means that following step 10) the temperature is lowered to 4°C.

Primers:
Pep481 AATTACGCGTGTTGGTGCGATAGTAGTAACG-3' (SEQ ID NO:6)
Pep482 TTAAGAATTCGAATGAAAGAGGAGGAATG-3' (SEQ ID NO:7)

The resulting 0.4kb PCR fragment from five positive strains (positive meaning giving DNA band of the right size) were purified and used in a DNA sequencing experiment, using once again as sequence primers the Pep481 (SEQ ID NO:6) and Pep482 (SEQ ID NO:7) primers.

Three of the five positive strains gave the same DNA sequence: Bacillus licheniformis ATCC 14580, Bacillus licheniformis NCIMB 6346 (=DSM 8785) and Bacillus licheniformis strain 712, resulting in the amino acid sequence of SEQ ID NO:2. In Bacillus licheniformis strain 470 DNA changes resulted in two amino acid changes (SEQ ID NO:9), however none in the mature peptide. In Bacillus licheniformis strain 009 DNA changes resulted in fifteen amino acid changes (SEQ ID NO:8), eight of which in the mature peptide. Furthermore, a consensus sequence (SEQ ID NO:10) was derived from SEQ ID NOs:2, 8 and 9.

Note that, in this experiment, the nucleotides encoding the seven C-terminal amino acids of SEQ ID NO:2 are included in the Pep481 primer (SEQ ID NO:6), and the seven C-terminal amino acid residues of SEQ ID NOs:8-9 may therefore not be correct. However the correctness of SEQ ID NOs:8-9 was later confirmed.

A strain of Bacillus licheniformis which was isolated from the in-feed probiotic product designated BioPlus™2B (offered by Chr. Hansen A/S, 10-12 Boege Allé, DK-2970 Hoersholm, Denmark) was included amongst the eleven strains tested but was negative.

In addition, 44 other strains of Bacillus licheniformis were tested as described above. A positive PCR-response was found in 27 of these strains. Examples of additional publicly available strains of Bacillus licheniformis found to be L12-positive have the following deposit numbers: NCTC 1024, NCTC 1025, NCTC 2120, NCTC 7589, NCTC 9932, ATCC 21424, NCIMB 10689, ATCC 53757. NCTC is the National Collection of Type Cultures. ATCC is the American Type Culture Collection. NCIMB is the National Collection of Industrial, Marine and Food Bacteria.

### Example 7: Thermostability

Differential Scanning Calorimetry (DSC) was used to determine temperature stability of the L12 protein at pH 2.5, 4.0 and 7.0. Purified L12 in a concentration of about 2 mg/ml was dialysed over night at 4°C against appropriate buffer and run on a VP-DSC instrument (MicroCal) with a constant scan rate of 1.5°C/min from 20 to 90°C. Data-handling was performed using the MicroCal Origin software (version 4.10), and denaturation temperature was defined as temperature at the apex of the enthalpy peak. In 10 mM citric acid, 50 mM sodium chloride, pH 2.5, L12 was found to have a denaturation temperature of 55°C. In 10 mM sodium acetate, 50 mM NaCl, pH 4.0, L12 denatured at 69°C, and in 10 mM sodium phosphate, 50 mM NaCl, pH 7.0 the denaturation temperature was 60°C.

### Example 8: Gut microflora modulation in vivo and in vitro

The influence of the L12 protein on the gut microflora was evaluated a) in vivo, in piglets and broilers; and b) in vitro, on microorganisms isolated from the gut microflora.

### Piglet study

This study, which was performed according to the French legal regulations on experiments with live animals, was performed to evaluate the effect of 5ppm of L12 (5 mg purified L12 per kg of feed) on piglet gut microflora.

Ten piglets (hybrids of Large-White, Landrace, and Piétrain, obtained from GAEC Leclerc, Ostheim, France) of an initial body weight of 25.3±1.3 kg were submitted to an ileo-rectal anastomosis (connecting the terminal ileum to the end of the rectum, bypassing the caecum and the colon). In such pigs, the microflora of the terminal ileum can be collected at the anus level and is representative of the bacterial population of all the consecutive digestive parts of the intestines. After surgery, during recovery from surgery, and during the experimental period the animals were placed in metabolic cages allowing an easy sampling of ileo-rectal contents.

During the experimental period of six weeks, the piglets were fed each and alternatively (in a double-latin-square design, to reduce the effect of individual variation and also any potential influence of the sequence of the treatments) a basal diet supplemented or not with test compounds. The diets were composed as follows:
Diet A: KLIBA, available from Provimi-Kliba, Kaiseraugst, Switzerland, with 18% soybean meal, 53% maize, 13% barley, 6% oat meal, 5.4% wheat bran, 1% soy oil, 3.6% minerals, vitamins and synthetic amino acids (w/w).
Diet E: Diet A with the addition of 5 mg/kg of the protein L12.
Diets B, C and D included other test compounds of no relevance for the present invention.

The test compounds (including L12) were incorporated into the diets in a Buhler mixer (Buhler, Aschwill, Switzerland). The experimental diets were prepared and administered to the animals in a mash form.

The experimental diets were allowed to the animals at the level of 2 kg per day distributed in two equal meals at 8:00 and 15:30.

Ileo-rectal contents were sampled from each animal at the two last days of each treatment period, and the concentrations of dry matter and of the different constituents of the microflora were determined.

The animals did not show any symptoms of toxicosis or of illness during the experiment. Their daily weight gain during the observation was 1.14+/-0.1 kg which is a very good zootechnical performance. At the end of the experiment the animals were euthanized by lethal injection after tranquilisation.

The dry matter contents of the samples were determined after drying overnight at 105°C, 1 g of sample, according to the standard Association of Official Analytical Chemists (AOAC) procedure (1009) (Association of Official Analytical Chemists. (1990). (Official methods of analysis. 15^{th} edition. Association of Official Analytical Chemists. Arlington.)

Analysis of the microflora was performed as follows:
Immediately after emission, 10g of ileo-rectal contents were transferred and homogenized into flasks containing 100 ml sodium chloride peptone broth (Merck, Darmstadt, Germany, catalogue no. 10582). Less than 5 min passed between emissions and removal of the ileal contents which were rapidly transferred to an anaerobic chamber (AES Cheminex, Combourg, France). Subsequently, the samples were serially diluted in 10-fold steps using sodium chloride peptone broth from 10⁻¹ to 10⁻⁸ (wt/vol). All bacterial counts were achieved with two replicate plates.

Total facultative anaerobic counts represent the average number of colonies that grew on Brucella agar (Merck, Darmstadt, Germany, catalogue no. 10490) supplemented with sheep blood (5% vol/vol, supplied by AES Cheminex, Combourg, France). Plates were incubated in an anaerobic cabinet at 37°C during 5 days.

Lactic acid bacteria were enumerated on MRS agar (Merck, Darmstadt, Germany, catalogue no. 110660) and Lactobacillus spp. on Rogosa agar (Merck, Darmstadt, Germany, catalogue no. 105413). Both plates were incubated in an anaerobic cabinet at 37°C during 48h.

Enterobacteriacae were counted on V.R.B.D agar (Merck, Darmstadt, Germany, catalogue no. 110275). Escherichia coli and other Enterobacteriacae were analyzed on a Coli-ID chromogenic media (BioMérieux, Marcy l'Etoile, France, catalogue no. 42017). This medium contains two chromogenic substrates: One for the detection of beta-D-glucuronidase (E.coli) producing pink colonies, and one for the detection of galactosidase (other Enterobacteriaceae) producing blue colonies. Both plates were incubated aerobically at 37°C during 24h.

Enterococcus spp. were evaluated on Enterococci agar (Merck, Darmstadt, Germany, catalogue no. 65009), and Staphylococcus spp. on Baird Parker agar (AES Cheminex, Combourg, France, catalogue no. AEB150302) after aerobic incubation at 37°C during 48h.

After heating the sample in a water bath at 80°C during 10 min, Clostridium perfringens was isolated using TSN agar (BioMérieux, Marcy l'Etoile, France, catalogue n° 51048) incubated in an anaerobic chamber at 46°C during 24 hours.

The identities of representative colonies were furthermore confirmed by microscopic examination after Gram staining and biochemical testing using the appropriate API system (BioMérieux, Marcy l'Etoile, France).

For each test group, statistic analysis of the data involved the calculation of the mean and standard deviation of the mean as well as an analysis of variance followed by Student's "t" test to assess the significance of inter-group differences.

The respective bacterial counts (number of colony forming units (CFU) per gram of ileal content dry matter (DM), +/- the standard deviation (SD) are presented in Table 3 below, which also shows the amendment in the respective bacterial counts caused by addition of 5 ppm L12, relative to the control.

**Table 3: Bacterial content of piglet Ileo-rectal microflora**

| **Bacterial count of:** | **Control (CFU/gDM)** | **5 ppm L12 (CFU/gDM)** | **Amendment (%)** | **Test t** |
|---|---|---|---|---|
| Facultative anaerobe bacteria | 2.78 x 10¹⁰ ± 1.94 x 10¹⁰ | 4.13 x 10¹⁰ ± 3.34 x 10¹⁰ | +49% | 0.15 |
| Total lactic acid bacteria | 1.21 x 10¹⁰ ± 1.28 x 10¹⁰ | 1.17 x 10¹⁰ ± 1.13 x 10¹⁰ | -4% | 0.91 |
| Lactobacillus spp. | 1.09 x 10¹⁰ ± 7.98 x 10⁹ | 1.04 x 10¹⁰ ± 8.15 x 10⁹ | -5% | 0.84 |
| Enterobacteriacae | 1.49 x 10⁸ ± 3.03 x 10⁸ | 1.47 x 10⁸ ± 2.04 x 10⁸ | -1% | 0.98 |
| Escherichia coli | 1.27 x 10⁸ ± 2.57 x 10⁸ | 1.51 x 10⁸ ± 2.40 x 10⁸ | +19% | 0.77 |
| Other Enterobacteriaceae | 1.05 x 10⁷ ± 2.66 x 10⁷ | 2.22 x 10⁵ ± 2.15 x 10⁵ | -98% | 0.31 |
| Enterococcus spp. | 3.89 x 10⁷ ± 6.82 x 10⁷ | 1.80 x 10⁷ ± 2.82 x 10⁷ | -54% | 0.22 |
| Staphylococcus spp. | 8.82 x 10⁵ ± 1.10 x 10⁶ | 7.86 x 10⁵ ± 1.29 x 10⁶ | -11% | 0.80 |
| Clostridium perfringens | 13 positives observations 20 2.99 x 10³ ± 8.51 x 10³ | 6 positives / observations / 20 2.59 x 10¹ ± 5.03 x 10¹ | -99% | 0.14 |

In this assay, no significant differences in the bacterial counts were found, only numeric effects were observed, but some times they were very strong.

A positive effect of L12 was seen on the mean of total facultative anaerobic bacteria, where the counts were increased by 49% relative to the control.

A neutral effect of L12 was seen on the total lactic acid bacteria and total Lactobacillus spp., which are representatives of the beneficial bacteria of the intestinal microbiota. The same is the case for total Enterobacteriaceae. The main component of the Enterobacteriaceae population in the piglet microflora, Escherichia coli, was increased by 19% whereas the group of other Enterobacteriaceae (other than E. coli) which are potentially pathogenic was strongly reduced.

A negative effect of L12 was seen on the population of Enterococcus spp.

Clostridium perfringens was found in thirteen of the twenty control samples (Diet A), whereas it was only found in six of the twenty samples with L12-supplemented diet (Diet E). This population of pathogenic bacteria was therefore strongly reduced (by 99%) with L12.

### Broiler study

In the broiler growth trial described in Example 5, the effect of L12 (5 mg purified L12 per kg of feed) on the broiler gut microflora was also evaluated.

Analysis of the microflora from twelve animals per group was performed as described above (the piglet study), using caecal content, after sacrifice of the broiler, as start material.

The results of the bacterial population counts in the broiler caecal contents are shown in Table 4.

**Table 4: Bacterial content of broiler caecal microflora**

| **Bacterial count of:** | **Control ( CFU / g DM )** | **5ppm L12 ( CFU / g DM )** | **Amendment (%)** | **Test t** |
|---|---|---|---|---|
| Facultative anaerobe bacteria | 1.40 x 10¹⁰ ± 1.09 x 10¹⁰ | 2.49 x 10¹⁰ ± 1.19 x 10¹⁰ | +178% | 0.03 |
| Total lactic acid bacteria | 2.15 x 10⁹ ± 2.31 x 10⁹ | 3.58 x 10⁹ ± 5.15 x 10⁹ | +166% | 0.39 |
| Escherichia coli | 1.64 x 10⁸ ± 1.64 x 10⁸ | 5.10 x 10⁸ ± 8.41 x 10⁸ | +311% | 0.19 |

A positive effect of L12 was seen in the total facultative anaerobe flora which increased statistically significantly by 178%.

This variation could be explained by the observed (numerical) increase of total lactic acid bacteria observed in the L12-supplemented group. Total lactic acid bacteria represent the main population of the total anaerobic facultative anaerobe flora and are composed mainly of Lactobacillus species which play an important role in the benefits imparted by the normal microbiota for the host health.

The counts of Escherichia coli were numerically, but not statistically significantly, increased in the L12-supplemented group. This is consistent with the increase in the counts of the other two groups of microorganisms. All the animals engaged in this experiment had healthy and equilibrated caecal microflora and no diarrheal disease in animals was observed during the trial, suggesting that it was commensal Escherichia coli strains which may have been increased.

### Gut microflora modulation in vitro

The effect of L12 on the microflora was further evaluated in vitro. Bacteria representative of beneficial and harmful microorganisms of the intestinal microbiota were isolated from piglet and broiler intestinal content. Their identities were confirmed by microscopic examination after Gram staining and biochemical testing using the appropriate API system (BioMérieux, Marcy l'Etoile, France).

The effect of L12 on the bacterial growth of these bacteria was determined by micro titre broth dilution method measuring absorbance at 595nm. All these in vitro assays were performed in sterilized 96-well plates (Falcon 353072 microtiter plates, Becton Dickinson Labware, Meylan, France) in a final volume of 110 microliter as follows: 100 microliter of suspension containing approximately 10⁵ CFU/ml of the pure bacteria in tryptic soy broth (Merck, Darmstadt, Germany, catalogue no. 105459) were added to 10 microliter of water containing L12 in serial 2-fold dilutions or to 10 microliter of water as control. Inhibition of growth was determined by measuring absorbance at 595nm with a Multiskan Ascent (ThermoLabsystems, Helsinki, Finland) after the appropriate time of incubation at the appropriate temperature for the optimal growth of the pure bacteria, see Table 5.

**Table 5: Incubation conditions**

| **Bacterial** **type** | **Bacterial strain** | **Condition of incubation** | **Temperature** **of** **incubation** | **Time of incubation** |
|---|---|---|---|---|
| Gram negative bacteria | All | Aerobe | 37°C | 24 hours |
| Gram | Enterococcus sp | Aerobe | 44°C | 48 hours |
| positive cocci | Staphylococcus sp | Aerobe | 37°C | 48 hours |
| Gram positive bacteria | Lactobacillus sp | Anaerobe | 37°C | 48 hours |
| | Clostridium perfringens | Anaerobe | 46°C | 24 hours |

The reduction in culture density was determined by subtracting the OD₅₉₅ of the test culture after 24 or 48 hours (according to Table 5) from the OD₅₉₅ of the control culture. Reduction in culture density was normalized by expressing it as a percentage of the OD₅₉₅ of the control culture and the MIC₉₀ corresponded to the concentration of L12 required to reduce culture density by 90%, meaning to inhibit the growth of 90% of the organisms tested (MIC₉₀ being defined as the Minimum Inhibitory Concentration required to inhibit the growth of 90% of organisms).

The results of these evaluations are shown in Table 6.

**Table 6: MIC₉₀ against bacteria isolated from piglet and broiler intestinal content.**

| **Bacterial type** | **Isolated from** | **Bacterial strain** | **MIC₉₀ (microgram/ml)** |
|---|---|---|---|
| Gram negative bacteria | Swine | Escherichia coli | > 1800 |
| | | Klebsiella pneumoniae | > 1800 |
| | | Salmonella enteritidis | > 1800 |
| | | Salmonella typhimurium | > 1800 |
| | Broiler | Escherichia coli | > 4170 |
| | | Proteus mirabilis | > 2085 |
| | | Salmonella enteritidis | > 4170 |
| | | Salmonella typhimurium | > 4170 |
| Gram positive cocci | Swine | Enterococcus faecalis | 33 |
| | | Enterococus faecium | 4 |
| | | Staphylococcus hyicus | 112 |
| | | Staphylococcus epidermidis | 450 |
| | Broiler | Enterococcus faecalis DSM 18047 | 65 |
| | | Enterococus durans | 33 |
| | | Staphylococcus xylosus | 1003 |
| Gram positive bacteria | Swine | Lactobacillus fermentum | > 1800 |
| | Broiler | Lactobacillus salivarius DSM 18070 | > 4170 |
| | | Clostridium perfringens | < 130 |

L12 is more active against Gram positive cocci and clostridia isolated from pig and broiler content than Gram negative bacteria isolated from same.

These results at least partially explained the influence of L12 on the gastro-intestinal microbiota:

The observed effect of L12 in vitro on Enterococcus faecalis and Enterococcus faecium confirmed the observed reduction in Enterococcus spp. observed in vivo in piglets.

The observed effect of L12 in vitro on Lactobacillus species confirmed the finding from the piglet and broiler in vivo studies that L12 has no negative influence in vivo on Lactobacillus species from swine and poultry.

The observed effect of L12 in vitro on Clostridium perfringens isolated from broiler intestinal content confirmed the strong reduction of this population observed in vivo in piglets.

### Example 9: Performance in animal feed in vivo (cage trial) - various dosages

The performance of the L12 protein in animal feed was evaluated in a broiler chicken growth trial with the following parameters:

| | |
|---|---|
| Growth trial: | Day 8 to day 29 (day 0 = day of hatch) |
| Treatments: | A: Control; B: 2.5, 5.0, and 7.5 mg purified L12 protein per kg feed |
| Diets: | Maize / SBM48 diet (see feed composition, Table 7) |

After mixing the feed was pelleted at about 70° C (3 x 25 mm). The L12 protein was dissolved in 500 ml of water and sprayed onto the pellets. For the control treatment the same quantity of water was sprayed onto the pellets.

| | |
|---|---|
| Replicates: | 10 groups of 6 male chicken, ROSS PM3, per treatment |
| Feeding: | Pellets ad libitum |

**Table 7: Feed composition of the experimental diet**

| Ingredients (%): | |
|---|---|
| Maize ⁴ | 62.94 |
| SBM 48 ⁵ | 30.00 |
| Soybean oil | 3.10 |
| DL-Methionine | 0.08 |
| Mono Calcium Phosphate | 1.50 |
| Limestone | 1.18 |
| Salt | 0.10 |
| TiO₂¹ | 0.10 |
| Premix no. UH 12699002 ² | 1.00 |

| Calculated content: | |
|---|---|
| Crude protein (%) | 19.0 |
| ME_{N} (MJ/kg) ³ | 12.7 |
| Crude fat (%) | 6.4 |
| Lysine (%) | 1.03 |
| Methionine (%) | 0.39 |
| Methionine + Cystine (%) | 0.71 |

| | |
|---|---|
| ¹ TiO₂ as indigestible marker was included in the feed mixture ² Commercially available from DSM Nutritional Products NV, Dorpsstraat 4, B-9800 Deinze, Belgium, and including Lasalocid Sodium , ³ Calculated with EC-equation (EEC (1986) Directive de la Commission du 9 avril 1986 fixant la méthode de calcul de la valeur énérgétique des aliments composés destinés à la volaille; Journal Officiel des Communautés Européennes, L 130, 53-54) ⁴ Raw material for feeding stuff; available from Moulin Moderne Hirsingue, Hirsingue, France ⁵ Soybean meal, residues of oil manufacture, raw material for feeding stuff; available from Moulin Moderne Hirsinque, Hirsinque, France | |

**Table 8: Performance of male broiler chicken; mean ± stdev**

| **Product** | **Control** | **L12 protein** | | |
|---|---|---|---|---|
| Dose (mg/kg feed) | 0 | 2.5 | 5.0 | 7.5 |
| Cages x birds | 10x6 | 10x6 | 10x6 | 10x6 |
| Weight gain (g/bird) | 1366 ^{A} | 1412 ^{A} | 1394 ^{A} | 1415 ^{A} |
| day 8-29 | ± 81 | ± 90 | ± 76 | ± 52 |
| % | *100.0* | *103.3* | *102.0* | *103.6* |
| Feed intake (g/bird) | 2210 ^{A} | 2214 ^{A} | 2195 ^{A} | 2207 ^{A} |
| day 8-29 | ± 96 | ± 89 | ± 91 | ± 55 |
| % | *100.0* | *100.2* | *99.3* | *99.9* |
| Feed conversion (g feed/g gain) | 1.619 ^{A} | 1.571 ^{B} | 1.576 ^{B} | 1.560 ^{B} |
| day 8-29 | ± 0.034 | ± 0.046 | ± 0.031 | ± 0.033 |
| *%* | *100.0* | *97.1* | *97.4* | *96.4* |

| | | | | |
|---|---|---|---|---|
| Newman-Keuls test: Means within a row, not sharing a common superscript, are significantly different (p < 0.05) | | | | |

### Example 10: Determination of concentration

A purified L12 protein preparation was used as an L12 protein standard. It was prepared as described in Example 4, and was formulated with glycerol as described in Example 2. The purity was above 96%, as measured by HPLC (using a Waters 2690 separation module and a Waters 2487 UV detector, detecting at 280 nm, using columns ACE C18 5µm 100A 150x3.0 mm and Waters µ-Bondapak C18 20x3.9 mm (guard column), a flow rate of 0.5 ml/min, an injection volume of 10 microliter, mobile phase A: H₂O 18MΩ + 0.1 % TFA (Tri-Fluor Acetic acid), mobile phase B Acetonitrile + 0.1 % TFA).

The L12-concentration of the standard was 3.6 mg pure protein/ml by, as determined by Amino Acid Analysis (as described below).

The purity and concentration of various other L12 samples were determined by an SDS-PAGE gel method as also described below, by reference to this standard.

### Amino Acid Analysis (AAA) - concentration of L12 protein standard

The peptide bonds of the L12 protein standard sample were subjected to acid hydrolysis, followed by separation and quantification of the released amino acids on a Biochrom 20 Plus Amino Acid Analyser, commercially available from Bie & Berntsen A/S, Sandbaekvej 5-7, DK-2610 Roedovre, Denmark, according to the manufacturer's instructions. For the acid hydrolysis, the protein sample was dried in a vacuum centrifuge, resolved in 18.5% (vol/vol) HCl + 0.1% (vol/vol) phenol and incubated for 16hr at 110°C. After incubation, the sample was again dried in the vacuum centrifuge, resolved in loading buffer (0.2 M Na-Citrate, pH 2.2) and loaded onto the Biochrom 20 Plus Amino Acid Analyser.

For the quantification, the hydrolysed sample was loaded onto a column of the cation-exchange resin UltroPac no. 8, Sodium-form, which is commercially available from Bie & Berntsen A/S, catalogue no. 80-2104-15. Buffers of varying pH (pH 1 to pH 8) and ionic strength were pumped through the column according to the manufacturer's instructions referred to above, to separate the various amino acids. The column temperature was accurately controlled, also according to the manufacturer's instructions (from 53°C to 92°C and back to 53°C) in order to ensure the required separation. The column eluent was mixed with ninhydrin reagent (Bie & Berntsen, catalogue no. 80-2038-07) and the mixture passed through the high temperature reaction coil of the Amino Acid Analyser. In the reaction coil, ninhydrin reacted with the amino acids to form coloured compounds, the amount of which was directly proportional to the quantity of amino acid present.

### Concentration of L12 protein samples

The concentration of various L12 samples were determined by the following procedure (all Novex products referred to are commercially available from Invitrogen, see www.invitrogen.com):

50 microliter L12 solution (0.1-1.0 mg L12 per ml) was mixed with 5 microliter 1% (w/v) EDTA + 10 microliter 6% PMSF + 10 microliter 0.5M DTT + 25 microliter NuPage LDS sample buffer (NP0007 from NOVEX) in an Eppendorf tube, and the tube was heated to 95°C for 5 minutes. 10 microliter sample was applied to a 10% Tris-Bis precast gel (NP0301 BOX from NOVEX). The electrophoresis was performed with a MES running buffer (MES=2-(N-morpholino)ethan sulfonic acid; NP0002 from NOVEX) + antioxidant (NP0005 from NOVEX) at a 200V constant voltage according to the manufacturer's instructions. After electrophoresis, the gel was gently shaken in 10% acetic acid + 50% EtOH for 10 minutes. The gel was then gently shaken with Colloidal Blue staining solution (46-7016 from NOVEX) for minimum three hours and washed by gentle shaking for 2 to 4 hours with distilled water with several changes of distilled water. The wet gel was scanned with a BioRad Calibrated Densitometer GS-800 equipped with Quantity One software (version 4.6.0, BioRad) and the L12 protein was quantified according to the manufacturer's instructions. The L12-standard described above was used as a standard, viz. in three-four dilutions within the range of 0.1-1.0 mg/ml.

### Example 11: Stability towards pepsin and pancreatin

Native L12: Purified L12 protein, diluted to 1 mg/ml in 10mM Tris/CH₃COOH buffer pH 4.5.

Denatured L12: A sample of purified L12 (1 mg/ml in 10mM Tris/CH₃COOH buffer pH 4.5) was boiled for 5 minutes in order to denature the protein.
Pepsin: Porcine pepsin (Sigma P-7000, 453 units/mg solid). 30000 units/ml were dissolved in 10mM succinic acid pH 3.0.
Pancreatin: Porcine pancreatin (Sigma P-7545, 8xUSP). 80 mg/ml were suspended in 0.5 M Na-phosphate buffer pH 7.0.
pH 3 buffer: 10mM succinic acid pH 3.0.
pH 7 buffer: 0.5 M Na-phosphate buffer pH 7.0.

The following samples were prepared:

### 1) L12 incubated with Pepsin and Pancreatin

278 microliter native or denatured L12 + 100 microliter pepsin solution + 122 microliter pH 3 buffer was incubated for 2 hours at 40°C with shaking, pH was measured to 3.5. Then 400 microliter pH 7 buffer + 100 microliter pancreatin suspension was added and followed by incubation for 4 hours at 40°C with shaking, pH was measured to 7.0.

### 2) L12 incubated with Pepsin

278 microliter native or denatured L12 + 100 microliter pepsin solution + 122 microliter pH 3 buffer was incubated for 2 hours at 40°C with shaking (pH 3.5 was measured). Then 500 microliter pH 7 buffer was added (pH 7.0 was measured).

### 3) L12 incubated with Pancreatin

278 microliter native or denatured L12 + 222 microliter pH 3 buffer was incubated for 2 hours at 40°C with shaking. Then 400 microliter pH 7 buffer + 100 microliter pancreatin suspension was added and followed by incubation for 4 hours at 40°C with shaking.

### 4) L12 control sample

278 microliter native or denatured L12 + 100 microliter pepsin solution + 122 microliter pH 3 buffer + 400 microliter pH 7 buffer + 100 microliter pancreatin suspension was mixed on ice and kept cold.

### 5) Pepsin control sample

100 microliter pepsin solution + 400 microliter pH 3 buffer was incubated for 2 hours at 40°C with shaking (pH 3.5 was measured). Then 500 microliter pH 7 buffer was added.

### 6) Pancreatin control sample

500 microliter pH 3 buffer + 400 microliter pH 7 buffer + 100 microliter pancreatin suspension was incubated for 4 hours at 40°C with shaking (pH 7.0 was measured).

### 7) Pepsin + Pancreatin control sample

100 microliter pepsin solution + 400 microliter pH 3 buffer was incubated for 2 hours at 40°C with shaking (pH 3.5 was measured). Then 400 microliter pH 7 buffer + 100 microliter pancreatin suspension was added and followed by incubation for 4 hours at 40°C with shaking (pH 7.0 was measured).

All samples were analyzed by SDS-PAGE as described in Example 10. Judged by SDS-PAGE, native L12 was not degraded by pepsin, pancreatin or pepsin-followed-by-pancreatin treatment. Denatured L12 was extensively degraded by pepsin, pancreatin or pepsin-followed-by-pancreatin treatment since no L12 band was detectable on the SDS-gel in the corresponding samples.

### Example 12: Animal feed performance in vivo (floor pen trial)

This example evaluates the effects of the L12 protein on the growth performance of broiler chickens over a full growth cycle of 36 days.

A growth performance trial with broiler chickens was performed from day 1 to day 36.

The animals were housed in floor pens littered with wood shavings. During the starter period (day 1-22) and during the grower period (day 22-36) the chickens received a diet based on wheat, rye and soybean meal (composition see Table 9). Beside an unsupplemented control treatment, L12 protein was included at a dosage of 7.5 mg protein per kg feed.

| | |
|---|---|
| Growth trial: | Day 1 to day 36 (day 0 = day of hatch) |
| Diets: | Wheat / rye / SBM48 diet (see feed composition in Table 9) |
| Feeding: | Pellets ad libitum |

After mixing, the feed was pelleted at about 70°C (3 x 25 mm). The L12 protein was dissolved in 800 ml of water for the starter feed and 1200 ml of water for the grower feed, respectively, and sprayed onto the pellets. For the control treatment the same quantity of water without the L12 protein was sprayed onto the pellets, respectively.

| | |
|---|---|
| Treatments: | Control, 7.5 mg L12 protein per kg feed |
| Replicates: | 6 groups of 20 chickens per sex and treatment (6 groups of 20 females, 6 groups of 20 males) |

**Table 9: Feed composition of the experimental diet**

| Ingredients (%): | Starter | Grower |
|---|---|---|
| Wheat⁴ | 41.20 | 46.00 |
| Rye⁴ | 15.00 | 15.00 |
| SBM 48⁵ | 34.30 | 30.73 |
| Soybean oil | 5.50 | 4.90 |
| DL-Methionine | 0.10 | 0.04 |
| DCP (Di-Calcium Phosphate) | 2.05 | 1.65 |
| Limestone | 0.65 | 0.43 |
| Salt | 0.20 | 0.15 |
| TiO₂ ¹ | -- | 0.10 |
| Premix no. 12699002² | 1.00 | 1.00 |

| Calculated content: | | |
|---|---|---|
| Crude protein (%) | 22.5 | 21.0 |
| ME_{N} (MJ/kg) ³ | 12.8 | 12.8 |
| Lysine (%) | 1.23 | 1.13 |
| Methionine (%) | 0.42 | 0.36 |
| Methionine + Cystine (%) | 0.91 | 0.73 |

| | | |
|---|---|---|
| ¹ TiO₂ as indigestible marker was included in the feed mixture ² Commercially available from DSM Nutritional Products NV, Dorpsstraat 4, B-9800 Deinze, Belgium, and including Lasalocid Sodium ³ Calculated with EC-equation equation (EEC (1986) Directive de la Commission du 9 avril 1986 fixant la méthode de calcul de la valeur énérgétique des aliments composés destinés à la volaille; Journal Officiel des Communautés Européennes, L 130, 53-54) ⁴ Raw material for feeding stuff; available from Moulin Moderne Hirsinque, Hirsingue, France ⁵ Soybean meal, residues of oil manufacture, raw material for feeding stuff; available from Moulin Moderne Hirsinque, Hirsinque, France | | |

**Table 10: Performance of broiler chickens; mean ± stdev**

| **Product** | **Control** | **L12 protein** |
|---|---|---|
| Treatment | A | B |
| Dose (mg/kg) | - | 7.5 |
| Number of pen x birds | 11* x 20 | 11* x 20 |
| Weight gain (g) | 2234 ^{A} | 2278 ^{A} |
| day 1-36 | ± 181 | ± 157 |
| (%) | 100.0 | 102.0 |
| Feed intake (g) | 3671 ^{A} | 3646 ^{A} |
| day 1-36 | ± 203 | ± 199 |
| (%) | 100.0 | 99.3 |
| Feed conversion (g feed/g gain) | 1.646 ^{A} | 1.602 ^{B} |
| day 1-36 | ± 0.045 | ± 0.047 |
| (%) | 100.0 | 97.3 |

| | | |
|---|---|---|
| Newman-Keuls test: Means within a row, not sharing a common superscript, are significantly different (p < 0.05) * one cage excluded due to technical problems | | |

The L12 protein improved the weight gain of the chickens slightly (Table 10), however this effect was not significant. The feed intake of the chickens receiving the L12 protein was not different to that of the control chickens. The feed conversion ratio (FCR) was significantly improved by the L12 protein compared to the control, viz. by 2.7%.

### Example 13: Animal feed and additive

### Animal Feed Additive

An animal feed additive is prepared by adding 25 g of a coated T-granulate comprising the purified L12 protein in an amount of 20 g/kg (prepared as described in Example 3 of EP 569468 B1, however with a coating of approx. 7% hydrogenated palm oil and approx. 13% CaCO₃) to the following premix (per kilo of premix):

| | |
|---|---|
| 1100000 IE | Vitamin A |
| 300000 IE | Vitamin D3 |
| 4000 IE | Vitamin E |
| 250 mg | Vitamin B1 |
| 800 mg | Vitamin B2 |
| 1200 mg | Ca-D-Panthothenate |
| 500 mg | Vitamin B6 |
| 2.5 mg | Vitamin B12 |
| 5000 mg | Niacin |
| 10000 mg | Vitamin C |
| 300 mg | Vitamin K3 |
| 15 mg | Biotin |
| 150 mg | Folic acid |
| 50004 mg | Cholin chloride |
| 6000 mg | Fe |
| 3000 mg | Cu |
| 5400 mg | Zn |
| 8000 mg | Mn |
| 124 mg | I |
| 60 mg | Co |
| 29.7 mg | Se |
| 9000 mg | Lasalocid Sodium (Avatec) |
| 17.3 % | Ca |
| 0.8 % | Mg |
| 11.7 % | Na |

### Animal Feed

A broiler grower diet having the following composition (%, w/w) is prepared by mixing the ingredients. Wheat, rye and SBM 48 are available from Moulin Moderne Hirsinque, Hirsingue, France. After mixing, the feed is pelleted at a desired temperature, e.g. about 70°C (3 x 25 mm).

| | |
|---|---|
| Wheat | 46.00 |
| Rye | 15.00 |
| Soy Bean Meal (SBM 48) | 30.73 |
| Soybean oil | 4.90 |
| DL-Methionine | 0.04 |
| DCP (Di-Calcium Phosphate) | 1.65 |
| Limestone | 0.43 |
| Salt | 0.15 |
| TiO₂ | 0.10 |
| Animal feed additive (above) | 1.00 |

The resulting animal feed comprises 5.0 mg purified L12 protein per kg (5ppm).

Additional animal feed and feed additive compositions are prepared in the same manner, however substituting 25 g coated L12 CT granulate per kg of the premix with 10¹³ Colony Forming Units (CFU), preferably in the form of endospores, of Bacillus licheniformis ATCC 14580, which results in an animal feed with approximately 10⁸ CFU per g of the feed composition.

## Claims

1. A nucleic acid construct comprising a polypeptide-encoding nucleic acid sequence, which polypeptide improves animal feed utilization by improving the feed conversion ratio (FCR), and which nucleic acid sequence
(a) hybridizes under medium stringency conditions with (i) nucleotides 124-378 of SEQ ID NO:1, a subsequence of (i) of at least 100 nucleotides, and/or (iii) a complementary strand of (i), or (ii); and/or
(b) has a degree of identity to nucleotides 124-378 of SEQ ID NO:1 of at least 48%, as determined by the align program using the default identity matrix, a penalty of -16 for the first residue of a gap and penalties of -4 for further residues of a gap;
with the proviso that the nucleic acid sequence is not SEQ ID NO:3, not nucleotides 601-978 of SEQ ID NO:3, and not nucleotides 1-381 of SEQ ID NO:1 the polynucleotide being operably linked to one or more control sequences that direct the production of the polypeptide in a Bacillus host cell, the DNA of which host cell, when harvested and used as a DNA template in a PCR reaction with SEQ ID NOs:6 and 7 as primers, leads to the generation of a PCR fragment of a size of approximately 0.4kb.

2. A recombinant expression vector comprising the nucleic acid construct of claim 1.

3. A recombinant Bacillus host cell comprising the nucleic acid construct of claim 1 or the vector of claim 2, the DNA of which host cell, when harvested and used as a DNA template in a PCR reaction with SEQ ID NOs:6 and 7 as primers, leads to the generation of a PCR fragment of a size of approximately 0.4kb.

4. A method for producing a polypeptide encoded by the polypeptide-encoding nucleic acid sequence of the construct of claim 1, which polypeptide improves animal feed utilization by improving the feed conversion ratio (FCR), the method comprising (a) cultivating a recombinant host cell of claim 3 to produce a supernatant comprising the polypeptide; and (b) recovering the polypeptide.

5. The method for producing a polypeptide of claim 4, the method comprising (a) cultivating a strain of Bacillus, the DNA of which, when harvested and used as a DNA template in a PCR reaction with SEQ ID NOs:6 and 7 as primers, leads to the generation of a PCR fragment of a size of approximately 0.4kb, which PCR fragment encodes an amino acid sequence which has at least 33% identity to amino acids 1-85 of SEQ ID NO:2, as determined by the Needle program, using the BLOSUM62 substitution matrix, a gap opening penalty of 10, and a gap extension penalty of 0.5; and (b) recovering the polypeptide.

6. Use in animal feed for non-therapeutic purposes of a polypeptide, which improves animal feed utilization by improving the feed conversion ratio (FCR), selected from the group consisting of:
(a) a polypeptide having amino acids 1-126 of SEQ ID NO:4;
(b) a polypeptide comprising an amino acid sequence which has a degree of identity to amino acids 1-85 of SEQ ID NO:2 of at least 33%, as determined by the Needle program, using the BLOSUM62 substitution matrix, a gap opening penalty of 10, and a gap extension penalty of 0.5; and
(c) a polypeptide which is encoded by a nucleic acid sequence which hybridizes under medium stringency conditions with (i) nucleotides 124-378 of SEQ ID NO:1, (ii) a subsequence of (i) of at least 100 nucleotides, and/or (iii) a complementary strand of (i), or (ii).

7. Use in the preparation of a composition for use in animal feed of a polypeptide, which improves animal feed utilization by improving the feed conversion ratio (FCR), selected from the group consisting of:
(a) a polypeptide having amino acids 1-126 of SEQ ID NO:4;
(b) a polypeptide comprising an amino acid sequence which has a degree of identity to amino acids 1-85 of SEQ ID NO:2 of at least 33%, as determined by the Needle program, using the BLOSUM62 substitution matrix, a gap opening penalty of 10, and a gap extension penalty of 0.5; and
(c) a polypeptide which is encoded by a nucleic acid sequence which hybridizes under medium stringency conditions with (i) nucleotides 124-378 of SEQ ID NO:1, (ii) a subsequence of (i) of at least 100 nucleotides, and/or (iii) a complementary strand of (i), or (ii).

8. A non-therapeutic method for improving animal feed conversion ratio (FCR), wherein a polypeptide, which improves animal feed utilization by improving the feed conversion ratio (FCR), is added to the feed, the polypeptide being selected from the group consisting of:
(a) a polypeptide having amino acids 1-126 of SEQ ID NO:4;
(b) a polypeptide comprising an amino acid sequence which has a degree of identity to amino acids 1-85 of SEQ ID NO:2 of at least 33%, as determined by the Needle program, using the BLOSUM62 substitution matrix, a gap opening penalty of 10, and a gap extension penalty of 0.5; and
(c) a polypeptide which is encoded by a nucleic acid sequence which hybridizes under medium stringency conditions with (i) nucleotides 124-378 of SEQ ID NO:1, (ii) a subsequence of (i) of at least 100 nucleotides, and/or (iii) a complementary strand of (i), or (ii).

9. An animal feed additive comprising a polypeptide, which improves animal feed utilization by improving the feed conversion ratio (FCR), and at least one additional component selected from the group consisting of fat-soluble vitamins, water-soluble vitamins, trace minerals, and macro minerals, wherein the polypeptide is selected from the group consisting of:
(a) a polypeptide having amino acids 1-126 of SEQ ID NO:4;
(b) a polypeptide comprising an amino acid sequence which has a degree of identity to amino acids 1-85 of SEQ ID NO:2 of at least 33%, as determined by the Needle program, using the BLOSUM62 substitution matrix, a gap opening penalty of 10, and a gap extension penalty of 0.5; and
(c) a polypeptide which is encoded by a nucleic acid sequence which hybridizes under medium stringency conditions with (i) nucleotides 124-378 of SEQ ID NO:1, (ii) a subsequence of (i) of at least 100 nucleotides, and/or (iii) a complementary strand of (i), or (ii).

10. An animal feed composition having a crude protein content of 50 to 800 g/kg and comprising a polypeptide, which improves animal feed utilization by improving the feed conversion ratio (FCR), selected from the group consisting of:
(a) a polypeptide having amino acids 1-126 of SEQ ID NO:4;
(b) a polypeptide comprising an amino acid sequence which has a degree of identity to amino acids 1-85 of SEQ ID NO:2 of at least 33%, as determined by the Needle program, using the BLOSUM62 substitution matrix, a gap opening penalty of 10, and a gap extension penalty of 0.5; and
(c) a polypeptide which is encoded by a nucleic acid sequence which hybridizes under medium stringency conditions with (i) nucleotides 124-378 of SEQ ID NO:1, (ii) a subsequence of (i) of at least 100 nucleotides, and/or (iii) a complementary strand of (i), or (ii).

## Patentansprüche

1. Nukleinsäurekonstrukt, umfassend eine Polypeptid-kodierende Nukleinsäuresequenz, wobei das Polypeptid die Tierfutternutzung durch Verbessern des Futterumsetzungsverhältnisses ("feed conversion ratio", FCR) verbessert, und wobei die Nukleinsäuresequenz
(a) unter mittleren Stringenzbedingungen mit (i) Nukleotiden 124-378 von SEQ ID NO: 1, (ii) einer Untersequenz von (i) von mindestens 100 Nukleotiden, und/oder (iii) einem komplementären Strang von (i) oder (ii) hybridisiert; und/oder
(b) einen Grad an Identität zu Nukleotiden 124-378 von SEQ ID NO: 1 von mindestens 48% hat, wie bestimmt durch das Align-Programm, unter Verwendung der vorgegebenen Identitätsmatrix, einem Strafwert von -16 für den ersten Rest einer Lücke und Strafwerten von -4 für weitere Reste einer Lücke;
mit der Maßgabe, dass die Nukleinsäuresequenz nicht SEQ ID NO: 3, nicht Nukleotide 601-978 von SEQ ID NO: 3 und nicht Nukleotide 1-381 von SEQ ID NO: 1 ist, wobei das Polynukleotid funktionsfähig mit einer oder mehreren Kontrollsequenzen, die die Herstellung des Polypeptids in einer Bacillus-Wirtszelle steuern, deren Wirtszell-DNA, wenn geerntet und als eine DNA-Matrize in einer PCR-Reaktion mit SEQ ID NO: 6 und 7 als Primern verwendet wird, zu der Bildung eines PCR-Fragments von einer Größe von ungefähr 0,4kb führt.

2. Rekombinanter Expressionsvektor, umfassend das Nukleinsäurekonstrukt gemäß Anspruch 1.

3. Rekombinante Bacillus-Wirtszelle, umfassend das Nukleinsäurekonstrukt gemäß Anspruch 1 oder den Vektor gemäß Anspruch 2, wobei die DNA der Wirtszelle, wenn geerntet und als eine DNA-Matrize in einer PCR-Reaktion mit SEQ ID NO: 6 und 7 als Primern verwendet, zu der Bildung eines PCR-Fragments von einer Größe von ungefähr 0,4kb führt.

4. Verfahren zum Herstellen eines Polypeptids, kodiert durch die Polypeptid-kodierende Nukleinsäuresequenz des Konstrukts gemäß Anspruch 1, wobei das Polypeptid die Tierfutternutzung durch Verbessern des Futterumsetzungsverhältnisses (FCR) verbessert, wobei das Verfahren umfasst (a) Kultivieren einer rekombinanten Wirtszelle gemäß Anspruch 3, zum Herstellen eines das Polypeptid umfassenden Überstands; und (b) Gewinnen des Polypeptids.

5. Verfahren zum Herstellen eines Polypeptids gemäß Anspruch 4, wobei das Verfahren umfasst (a) Kultivieren eines Stamms von Bacillus, dessen DNA, wenn geerntet und als eine DNA-Matrize in einer PCR-Reaktion mit SEQ ID NO: 6 und 7 als Primer verwendet, zu der Bildung eines PCR-Fragmentes von einer Größe von ungefähr 0,4kb führt, wobei das PCR-Fragment eine Aminosäuresequenz kodiert, die mindestens 33% Identität zu Aminosäuren 1-85 von SEQ ID NO: 2 hat, wie bestimmt durch das Needle-Programm, unter Verwendung der BLOSUM62-Substitutionsmatrix, einem Lückenöffnungsstrafwert von 10 und einem Lückenfortsetzungsstrafwert von 0,5; und (b) Gewinnen des Polypeptids.

6. Verwendung eines Polypeptids in Tierfutter fiir nicht therapeutische Zwecke, das die Tierfutternutzung durch Verbessern des Futterumsetzungsverhältnisses (FCR) verbessert, ausgewählt aus der Gruppe bestehend aus:
(a) einem Polypeptid mit Aminosäuren 1-126 von SEQ ID NO: 4;
(b) einem Polypeptid, umfassend eine Aminosäuresequenz, die einen Grad an Identität zu Aminosäuren 1-85 von SEQ ID NO: 2 von mindestens 33% hat, wie bestimmt durch das Needle-Programm, unter Verwendung der BLOSUM62-Substitutionsmatrix, einem Lückenöffnungsstrafwert von 10 und einem Lückenverlängerungsstrafwert von 0,5; und
(c) einem Polypeptid, das durch eine Nukleinsäuresequenz kodiert ist, die unter mittleren Stringenzbedingungen mit (i) Nukleotiden 124-378 von SEQ ID NO:1, (ii) einer Untersequenz von (i) von mindestens 100 Nukleotiden und/oder (iii) einem komplementären Strang von (i) oder (ii) hybridisiert.

7. Verwendung eines Polypeptids in der Herstellung einer Zusammensetzung zur Verwendung in Tierfutter, welches die Tierfutternutzung durch Verbessern des Futterumsetzungsverhältnisses (FCR) verbessert, ausgewählt aus der Gruppe bestehend aus:
(a) einem Polypeptid mit Aminosäuren 1-126 von SEQ ID NO: 4;
(b) einem Polypeptid, umfassend eine Aminosäuresequenz, die einen Grad an Identität zu Aminosäuren 1-85 von SEQ ID NO: 2 von mindestens 33% hat, wie bestimmt durch das Needle-Programm, unter Verwendung der BLOSUM62-Substitutionsmatrix, einem Lückenöffnungsstrafwert von 10 und einem Lückenverlängerungsstrafwert von 0,5; und
(c) einem Polypeptid, das durch eine Nukleinsäuresequenz kodiert ist, die unter mittleren Stringenzbedingungen mit (i) Nukleotiden 124-378 von SEQ ID NO:1, (ii) einer Untersequenz von (i) von mindestens 100 Nukleotiden und/oder (iii) einem komplementären Strang von (i) oder (ii) hybridisiert.

8. Nicht-therapeutisches Verfahren zum Verbessern des Tierfutterumsetzungsverhältnisses (FCR), wobei ein Polypeptid, das die Tierfutternutzung durch Verbessern des Futterumsetzungsverhältnisses (FCR) verbessert, zu dem Futter hinzugefügt wird, wobei das Polypeptid ausgewählt ist aus der Gruppe bestehend aus:
(a) einem Polypeptid mit Aminosäuren 1-126 von SEQ ID NO: 4;
(b) einem Polypeptid, umfassend eine Aminosäuresequenz, die einen Grad an Identität zu Aminosäuren 1-85 von SEQ ID NO: 2 von mindestens 33% hat, wie bestimmt durch das Needle-Programm, unter Verwendung der BLOSUM62-Substitutionsmatrix, einem Lückenöffnungsstrafwert von 10 und einem Lückenverlängerungsstrafwert von 0,5; und
(c) einem Polypeptid, das durch eine Nukleinsäuresequenz kodiert ist, die unter mittleren Stringenzbedingungen mit (i) Nukleotiden 124-378 von SEQ ID NO: 1, (ii) einer Untersequenz von (i) von mindestens 100 Nukleotiden und/oder (iii) einem komplementären Strang von (i) oder (ii) hybridisiert.

9. Tierfutteradditiv, umfassend ein Polypeptid, welches die Tierfutternutzung durch Verbessern des Futterumsetzungsverhältnisses (FCR) verbessert, und mindestens einen zusätzlichen Bestandteil, ausgewählt aus der Gruppe bestehend aus fettlöslichen Vitaminen, wasserlöslichen Vitaminen, Spurenmineralien und Makromineralien, wobei das Polypeptid ausgewählt ist aus der Gruppe bestehend aus:
(a) einem Polypeptid mit Aminosäuren 1-126 von SEQ ID NO: 4;
(b) einem Polypeptid, umfassend eine Aminosäuresequenz, die einen Grad an Identität zu Aminosäuren 1-85 von SEQ ID NO: 2 von mindestens 33% hat, wie bestimmt durch das Needle-Programm, unter Verwendung der BLOSUM62-Substitutionsmatrix, einem Lückenöffnungsstrafwert von 10 und einem Lückenverlängerungsstrafwert von 0,5; und
(c) einem Polypeptid, das durch eine Nukleinsäuresequenz kodiert ist, die unter mittleren Stringenzbedingungen mit (i) Nukleotiden 124-378 von SEQ ID NO: 1, (ii) einer Untersequenz von (i) von mindestens 100 Nukleotiden, und/oder (iii) einem komplementären Strang von (i) oder (ii) hybridisiert.

10. Tierfutterzusammensetzung mit einem Rohproteingehalt von 50-800 g/kg und umfassend ein Polypeptid, welches die Tierfutternutzung durch Verbessern des Futterumsetzungsverhältnisses (FCR) verbessert, ausgewählt aus der Gruppe bestehend aus:
(a) einem Polypeptid mit Aminosäuren 1-126 von SEQ ID NO: 4;
(b) einem Polypeptid, umfassend eine Aminosäuresequenz, die einen Grad an Identität zu Aminosäuren 1-85 von SEQ ID NO: 2 von mindestens 33% hat, wie bestimmt durch das Needle-Programm, unter Verwendung der BLOSUM62-Substitutionsmatrix, einem Lückenöffnungsstrafwert von 10 und einem Lückenverlängerungsstrafwert von 0,5; und
(c) einem Polypeptid, das durch eine Nukleinsäuresequenz kodiert ist, die unter mittleren Stringenzbedingungen mit (i) Nukleotiden 124-378 von SEQ ID NO: 1, (ii) einer Untersequenz von (i) von mindestens 100 Nukleotiden, und/oder (iii) einem komplementären Strang von (i) oder (ii) hybridisiert.

## Revendications

1. Construction d'acide nucléique comprenant une séquence d'acide nucléique codant pour un polypeptide, lequel polypeptide améliore l'utilisation des aliments pour animaux en améliorant l'indice de consommation (FCR), et laquelle séquence d'acide nucléique
(a) s'hybride dans des conditions de stringence moyennes (i) aux nucléotides 124 à 378 de SEQ ID NO : 1, (ii) à une sous-séquence de (i) d'au moins 100 nucléotides et/ou (iii) à un brin complémentaire de (i) ou (ii) ; et/ou
(b) présente un degré d'identité avec les nucléotides 124 à 378 de SEQ ID NO : 1 d'au moins 48 %, déterminé par le programme d'alignement en utilisant la matrice d'identité par défaut, une pénalité de -16 pour le premier résidu d'une brèche et des pénalités de -4 pour d'autres résidus d'une brèche ;
à condition que la séquence d'acide nucléique ne soit pas SEQ ID NO : 3, les nucléotides 601 à 978 de SEQ ID NO : 3 et les nucléotides 1 à 381 de SEQ ID NO : 1, le polynucléotide étant lié de manière fonctionnelle à une ou plusieurs séquences de contrôle qui dirigent la production du polypeptide dans une cellule hôte de Bacillus, l'ADN de ladite cellule hôte, lorsqu'il est récolté et utilisé en tant que matrice d'ADN dans une réaction de PCR avec SEQ ID NO : 6 et 7 comme amorces, mène à la génération d'un fragment de PCR d'une taille d'approximativement 0,4 kb.

2. Vecteur d'expression recombinant comprenant la construction d'acide nucléique selon la revendication 1.

3. Cellule hôte de Bacillus recombinante comprenant la construction d'acide nucléique selon la revendication 1 ou le vecteur selon la revendication 2, l'ADN de ladite cellule hôte, lorsqu'il est récolté et utilisé en tant que matrice d'ADN dans une réaction de PCR avec SEQ ID NO : 6 et 7 comme amorces, mène à la génération d'un fragment de PCR d'une taille d'approximativement 0,4 kb.

4. Procédé de production d'un polypeptide codé par la séquence d'acide nucléique codant pour un polypeptide de la construction selon la revendication 1, lequel polypeptide améliore l'utilisation des aliments pour animaux en améliorant l'indice de consommation (FCR), le procédé comprenant (a) la culture d'une cellule hôte recombinante selon la revendication 3 pour produire un surnageant comprenant le polypeptide ; et (b) la récupération du polypeptide.

5. Procédé de production d'un polypeptide selon la revendication 4, le procédé comprenant (a) la culture d'une souche de Bacillus, dont l'ADN, lorsqu'il est récolté et utilisé en tant que matrice d'ADN dans une réaction de PCR avec SEQ ID NO : 6 et 7 comme amorces, mène à la génération d'un fragment de PCR d'une taille d'approximativement 0,4 kb, lequel fragment de PCR code pour une séquence d'acides aminés qui présente au moins 33 % d'identité avec les acides aminés 1 à 85 de SEQ ID NO : 2, déterminée par le programme Needle, en utilisant la matrice de substitution BLOSUM62, une pénalité d'ouverture de brèche de 10 et une pénalité d'extension de brèche de 0,5 ; et (b) la récupération du polypeptide.

6. Utilisation dans des aliments pour animaux pour des objectifs non thérapeutiques d'un polypeptide qui améliore l'utilisation des aliments pour animaux en améliorant l'indice de consommation (FCR), choisi dans le groupe constitué par :
(a) un polypeptide ayant les acides aminés 1 à 126 de SEQ ID NO : 4 ;
(b) un polypeptide comprenant une séquence d'acides aminés qui présente un degré d'identité avec les acides aminés 1 à 85 de SEQ ID NO : 2 d'au moins 33 %, déterminé par le programme Needle, en utilisant la matrice de substitution BLOSUM62, une pénalité d'ouverture de brèche de 10 et une pénalité d'extension de brèche de 0,5 ; et
(c) un polypeptide qui est codé par une séquence d'acide nucléique qui s'hybride dans des conditions de stringence moyennes (i) aux nucléotides 124 à 378 de SEQ ID NO : 1, (ii) à une sous-séquence de (i) d'au moins 100 nucléotides et/ou (iii) à un brin complémentaire de (i) ou (ii).

7. Utilisation dans la préparation d'une composition en vue d'une utilisation dans des aliments pour animaux d'un polypeptide qui améliore l'utilisation des aliments pour animaux en améliorant l'indice de consommation (FCR), choisi dans le groupe constitué par :
(a) un polypeptide ayant les acides aminés 1 à 126 de SEQ ID NO : 4 ;
(b) un polypeptide comprenant une séquence d'acides aminés qui présente un degré d'identité avec les acides aminés 1 à 85 de SEQ ID NO : 2 d'au moins 33 %, déterminé par le programme Needle, en utilisant la matrice de substitution BLOSUM62, une pénalité d'ouverture de brèche de 10 et une pénalité d'extension de brèche de 0,5 ; et
(c) un polypeptide qui est codé par une séquence d'acide nucléique qui s'hybride dans des conditions de stringence moyennes (i) aux nucléotides 124 à 378 de SEQ ID NO : 1, (ii) à une sous-séquence de (i) d'au moins 100 nucléotides et/ou (iii) à un brin complémentaire de (i) ou (ii).

8. Procédé non thérapeutique permettant d'améliorer l'indice de consommation (FCR), dans lequel un polypeptide qui améliore l'utilisation des aliments pour animaux en améliorant l'indice de consommation (FCR), est ajouté aux aliments, le polypeptide étant choisi dans le groupe constitué par :
(a) un polypeptide ayant les acides aminés 1 à 126 de SEQ ID NO : 4 ;
(b) un polypeptide comprenant une séquence d'acides aminés qui présente un degré d'identité avec les acides aminés 1 à 85 de SEQ ID NO : 2 d'au moins 33 %, déterminé par le programme Needle, en utilisant la matrice de substitution BLOSUM62, une pénalité d'ouverture de brèche de 10 et une pénalité d'extension de brèche de 0,5 ; et
(c) un polypeptide qui est codé par une séquence d'acide nucléique qui s'hybride dans des conditions de stringence moyennes (i) aux nucléotides 124 à 378 de SEQ ID NO : 1, (ii) à une sous-séquence de (i) d'au moins 100 nucléotides et/ou (iii) à un brin complémentaire de (i) ou (ii).

9. Additif d'aliments pour animaux comprenant un polypeptide qui améliore l'utilisation des aliments pour animaux en améliorant l'indice de consommation (FCR), et au moins un composant supplémentaire choisi dans le groupe constitué par des vitamines solubles dans les graisses, des vitamines hydrosolubles, des oligominéraux et des macrominéraux, où le polypeptide est choisi dans le groupe constitué par :
(a) un polypeptide ayant les acides aminés 1 à 126 de SEQ ID NO : 4 ;
(b) un polypeptide comprenant une séquence d'acides aminés qui présente un degré d'identité avec les acides aminés 1 à 85 de SEQ ID NO : 2 d'au moins 33 %, déterminé par le programme Needle, en utilisant la matrice de substitution BLOSUM62, une pénalité d'ouverture de brèche de 10 et une pénalité d'extension de brèche de 0,5 ; et
(c) un polypeptide qui est codé par une séquence d'acide nucléique qui s'hybride dans des conditions de stringence moyennes (i) aux nucléotides 124 à 378 de SEQ ID NO : 1, (ii) à une sous-séquence de (i) d'au moins 100 nucléotides et/ou (iii) à un brin complémentaire de (i) ou (ii).

10. Composition d'aliments pour animaux présentant une teneur brute en protéines de 50 à 800 g/kg et comprenant un polypeptide qui améliore l'utilisation des aliments pour animaux en améliorant l'indice de consommation (FCR), choisi dans le groupe constitué par :
(a) un polypeptide ayant les acides aminés 1 à 126 de SEQ ID NO : 4 ;
(b) un polypeptide comprenant une séquence d'acides aminés qui présente un degré d'identité avec les acides aminés 1 à 85 de SEQ ID NO : 2 d'au moins 33 %, déterminé par le programme Needle, en utilisant la matrice de substitution BLOSUM62, une pénalité d'ouverture de brèche de 10 et une pénalité d'extension de brèche de 0,5 ; et
(c) un polypeptide qui est codé par une séquence d'acide nucléique qui s'hybride dans des conditions de stringence moyennes (i) aux nucléotides 124 à 378 de SEQ ID NO : 1, (ii) à une sous-séquence de (i) d'au moins 100 nucléotides et/ou (iii) à un brin complémentaire de (i) ou (ii).


### SEQUENCE LISTING

<110> Novozymes A/S
<120> Polypeptides
<130> 10799.204-WO
<160> 10
<170> PatentIn version 3.3
<210> 1
   <211> 381
   <212> DNA
   <213> Bacillus licheniformis ATCC 14580
<220>
   <221> CDS
   <222> (1)..(378)
<220>
   <221> mat_peptide
   <222> (124)..(378)
<400> 1
   atg aaa aat cat ttg tat gag aaa aaa aag agg aaa cct ttg act cgg 48
   Met Lys Asn His Leu Tyr Glu Lys Lys Lys Arg Lys Pro Leu Thr Arg
   -40 -35 -30
   aca att aaa gcg acg ctc gcc gtg ttg aca atg tcc atc gct ttg gtg 96
   Thr Ile Lys Ala Thr Leu Ala Val Leu Thr Met Ser Ile Ala Leu Val
   -25 -20 -15 -10
   gga ggc gct acg gtg cct tca ttt gca tgg gtg aat ccg ggt tat cac 144
   Gly Gly Ala Thr Val Pro Ser Phe Ala Trp Val Asn Pro Gly Tyr His
   -5 -1 1 5
   tac cag tac cca tcg gaa ggt ggt aca tgg agg tat gga ttc gta aac 192
   Tyr Gln Tyr Pro Ser Glu Gly Gly Thr Trp Arg Tyr Gly Phe Val Asn
   10 15 20
   gcc ggg ctc cgt tca gag tac aac cac ccg aca aag gtc cac ggc tcg 240
   Ala Gly Leu Arg Ser Glu Tyr Asn His Pro Thr Lys Val His Gly Ser
   25 30 35
   aca gtg caa aag ctc atc gat gga aaa gtg gat aaa acg aat aga agt 288
   Thr Val Gln Lys Leu Ile Asp Gly Lys Val Asp Lys Thr Asn Arg Ser
   40 45 50 55
   att gat acg gct gcg ggc cgc tac tct aat gcc tat gtc gga gcc ata 336
   Ile Asp Thr Ala Ala Gly Arg Tyr Ser Asn Ala Tyr Val Gly Ala Ile
   60 65 70
   aac tca cct ggt ctt aag ggt cgt tac tac tat cgc acc aac taa 381
   Asn Ser Pro Gly Leu Lys Gly Arg Tyr Tyr Tyr Arg Thr Asn
   75 80 85
<210> 2
   <211> 126
   <212> PRT
   <213> Bacillus licheniformis ATCC 14580
<400> 2
   Met Lys Asn His Leu Tyr Glu Lys Lys Lys Arg Lys Pro Leu Thr Arg
       -40                 -35                 -30
   Thr Ile Lys Ala Thr Leu Ala Val Leu Thr Met Ser Ile Ala Leu Val
   -25                 -20                 -15                 -10
   Gly Gly Ala Thr Val Pro Ser Phe Ala Trp Val Asn Pro Gly Tyr His
                   -5              -1  1               5
   Tyr Gln Tyr Pro Ser Glu Gly Gly Thr Trp Arg Tyr Gly Phe Val Asn
           10                  15                  20
   Ala Gly Leu Arg Ser Glu Tyr Asn His Pro Thr Lys Val His Gly Ser
       25                  30                  35
   Thr Val Gln Lys Leu Ile Asp Gly Lys Val Asp Lys Thr Asn Arg Ser
   40                  45                  50                  55
   Ile Asp Thr Ala Ala Gly Arg Tyr Ser Asn Ala Tyr Val Gly Ala Ile
                   60                  65                  70
   Asn Ser Pro Gly Leu Lys Gly Arg Tyr Tyr Tyr Arg Thr Asn
               75                  80                  85
<210> 3
   <211> 1581
   <212> DNA
   <213> Bacillus licheniformis
<220>
   <221> CDS
   <222> (601)..(978)
<400> 3
   gaattttccg gaagctgaaa cacccgtgat atatataacc ataaattaaa cagcataggc      60
   ggattgtgcg agttcctcca cattcggagt atttctgaat gatagagcca cacggtccac     120
   gttctcactg gctaaccgga tcaaatgatc ttcaggagtc agcataatac atccagttca     180
   ggtagataag atttgaattt ggtgacttgc ttttgttctt cttctttcat tttctgacta     240
   atccaaactg gaaaaagcag gtcttttaac agattaggag gtttctgaca tgcaccattc     300
   ggtcactaac cgaatgcagt aaaggacact gtggtgcttg ccagccatta gggtattgag     360
   gaggtgatca aaatgctagg tgacagtatt tcgtcgaagt ggacaagtcg tgaccaaatg     420
   acctcggatc gagggttggt catggaggaa aaaattgatg tctggtgaca aagaggagtc     480
   atgatcatgg caccgccaac gagggaaaaa actcttcccg catcgacacg gtatgtgggc     540
   ggtgacaaac taacttatag agtaaattta ttagtcgaat gaaagaggag gaatgaaata     600
   atg aaa aat cat ttg tat gag aaa aaa aag agg aaa cct ttg act cgg       648
   Met Lys Asn His Leu Tyr Glu Lys Lys Lys Arg Lys Pro Leu Thr Arg
   1               5                   10                  15
   aca att aaa gcg acg ctc gcc gtg ttg aca atg tcc atc gct ttg gtg       696
   Thr Ile Lys Ala Thr Leu Ala Val Leu Thr Met Ser Ile Ala Leu Val
                   20                       25                        30
   gga ggc gct acg gtg cct tca ttt gca tgg gtg aat ccg ggt tat cac       744
   Gly Gly Ala Thr Val Pro Ser Phe Ala Trp Val Asn Pro Gly Tyr His
           35                  40                      45
   tac cag tac cca tcg gaa ggt ggt aca tgg agg tat gga ttc gta aac       792
   Tyr Gln Tyr Pro Ser Glu Gly Gly Thr Trp Arg Tyr Gly Phe Val Asn
       50                  55                      60
   gcc ggg ctc cgt tca gag tac aac cac ccg aca aag gtc cac ggc tcg       840
   Ala Gly Leu Arg Ser Glu Tyr Asn His Pro Thr Lys Val His Gly Ser
   65                  70                  75                  80
   aca gtg caa aag ctc atc gat gga aaa gtg gat aaa acg aat aga agt       888
   Thr Val Gln Lys Leu Ile Asp Gly Lys Val Asp Lys Thr Asn Arg Ser
                   85                  90                  95
   att gat acg gct gcg ggc cgc tac tct aat gcc tat gtc gga gcc ata       936
   Ile Asp Thr Ala Ala Gly Arg Tyr Ser Asn Ala Tyr Val Gly Ala Ile
               100                 105                 110
   aac tca cct ggt ctt aag ggt cgt tac tac tat cgc acc aac               978
   Asn Ser Pro Gly Leu Lys Gly Arg Tyr Tyr Tyr Arg Thr Asn
           115                 120                 125
   taatcaaagg gaaaacggtt gctgtcaacg gggctagcat ggcaagaccc agaaaagttc    1038
   tgggagatcc cgctttgcat aagcgtatta tagtggatga cgcgggcttt gttgtttaca    1098
   cttcttgcac ctgctgacgg caatcatccc tatctatgaa atcgagattt cagcaggccg    1158
   ttattttcga gagagttaaa tctatattca ttgtttttat tttggtaagg acataccgga    1218
   ttttaggttt ggattaccgg tcgagttagc ttgtcttttc gcccactacc gtgtcgatgc    1278
   gggagcaatt taccagaagc acttaccgat tgatagtttt ttattccggt gattgcaaag    1338
   tttcataaac tctgagaatt caataggggt aataccccgc tttgaggggc gcggcatttt    1398
   atgcgccccg agtatttatt cttaaaattt ttaaattaat gtatctatat aaaaaggaga    1458
   tgctttcggt gtactgccaa agcatctcca caaaagatag tgcatatctg caggaaaaaa    1518
   cataaaatgc aactaacatt tttttggaaa gcaataggtt tatttaattt tgtagtttta    1578
   tct                                                                  1581
<210> 4
   <211> 126
   <212> PRT
   <213> Bacillus licheniformis
<400> 4
   Met Lys Asn His Leu Tyr Glu Lys Lys Lys Arg Lys Pro Leu Thr Arg
   1               5                   10                  15
   Thr Ile Lys Ala Thr Leu Ala Val Leu Thr Met Ser Ile Ala Leu Val
               20                  25                  30
   Gly Gly Ala Thr Val Pro Ser Phe Ala Trp Val Asn Pro Gly Tyr His
           35                  40                  45
   Tyr Gln Tyr Pro Ser Glu Gly Gly Thr Trp Arg Tyr Gly Phe Val Asn
       50                  55                  60
   Ala Gly Leu Arg Ser Glu Tyr Asn His Pro Thr Lys Val His Gly Ser
   65                  70                  75                  80
   Thr Val Gln Lys Leu Ile Asp Gly Lys Val Asp Lys Thr Asn Arg Ser
                   85                  90                  95
   Ile Asp Thr Ala Ala Gly Arg Tyr Ser Asn Ala Tyr Val Gly Ala Ile
               100                 105                 110
   Asn Ser Pro Gly Leu Lys Gly Arg Tyr Tyr Tyr Arg Thr Asn
           115                 120                 125
<210> 5
   <211> 15
   <212> PRT
   <213> Bacillus licheniformis ATCC 14580
<220>
   <221> MISC_FEATURE
   <223> N-terminal
<400> 5
   Trp Val Asn Pro Gly Tyr His Tyr Gln Tyr Pro Ser Glu Gly Gly
   1               5                   10                  15
<210> 6
   <211> 31
   <212> DNA
   <213> Artificial
<220>
   <223> Primer Pep 481
<220>
   <221> misc_feature
   <223> Primer
<400> 6
   aattacgcgt gttggtgcga tagtagtaac g 31
<210> 7
   <211> 29
   <212> DNA
   <213> Artificial
<220>
   <223> Primer Pep 482
<220>
   <221> misc_feature
   <223> Primer
<400> 7
   ttaagaattc gaatgaaaga ggaggaatg 29
<210> 8
   <211> 125
   <212> PRT
   <213> Bacillus licheniformis strain 009
<220>
   <221> mat_peptide
   <222> (41)..(125)
<400> 8
   Met Lys Asn Leu Leu Asn Lys Lys Lys Arg Lys Pro Leu Thr Arg Thr
   -40                 -35                 -30                 -25
   Ile Lys Ala Thr Phe Ala Val Leu Thr Val Ser Ile Gly Leu Val Gly
                   -20                 -15                 -10
   Gly Ala Thr Val Pro Ala Phe Ala Trp Val Asn Pro Asp Tyr His Tyr
               -5              -1  1               5
   Gln Tyr Pro Ser Glu Gly Gly Thr Trp Arg Tyr Gly Phe Val Asn Leu
       10                  15                  20
   Gly Leu Arg Ser Glu Tyr Asn His Pro Lys Lys Val His Gly Ser Thr
   25                  30                  35                  40
   Val Gln Lys Leu Ile Asp Gly Lys Val Glu Lys Thr Asn Arg Ser Leu
                   45                  50                  55
   Asp Thr Ala Pro Gly Arg Tyr Ser Asn Ala Tyr Val Gly Val Val Asn
               60                  65                  70
   Ser Pro Gly Leu Lys Gly Arg Tyr Tyr Tyr Arg Thr Asn
           75                  80                  85
<210> 9
   <211> 126
   <212> PRT
   <213> Bacillus licheniformis strain 470
<220>
   <221> mat_peptide
   <222> (42)..(126)
<400> 9
   Met Lys Asn Tyr Leu Tyr Glu Lys Lys Lys Arg Lys Pro Leu Thr Arg
       -40                 -35                 -30
   Thr Ile Lys Ala Thr Leu Ala Val Leu Thr Met Ser Ile Ala Leu Val
   -25                 -20                 -15                 -10
   Gly Gly Ala Thr Val Pro Ala Phe Ala Trp Val Asn Pro Gly Tyr His
                   -5                  -1  1               5
   Tyr Gln Tyr Pro Ser Glu Gly Gly Thr Trp Arg Tyr Gly Phe Val Asn
           10                  15                  20
   Ala Gly Leu Arg Ser Glu Tyr Asn His Pro Thr Lys Val His Gly Ser
       25                  30                  35
   Thr Val Gln Lys Leu Ile Asp Gly Lys Val Asp Lys Thr Asn Arg Ser
   40                  45                  50                  55
   Ile Asp Thr Ala Ala Gly Arg Tyr Ser Asn Ala Tyr Val Gly Ala Ile
                   60                  65                  70
   Asn Ser Pro Gly Leu Lys Gly Arg Tyr Tyr Tyr Arg Thr Asn
               75                  80                  85
<210> 10
   <211> 126
   <212> PRT
   <213> Artificial
<220>
   <223> Consensus sequence
<220>
   <221> mat_peptide
   <222> (42)..(126)
<400> 10
   Met Lys Asn His Leu Tyr Glu Lys Lys Lys Arg Lys Pro Leu Thr Arg
       -40                 -35                 -30
   Thr Ile Lys Ala Thr Leu Ala Val Leu Thr Met Ser Ile Ala Leu Val
   -25                 -20                 -15                 -10
   Gly Gly Ala Thr Val Pro Ala Phe Ala Trp Val Asn Pro Gly Tyr His
                   -5              -1  1               5
   Tyr Gln Tyr Pro Ser Glu Gly Gly Thr Trp Arg Tyr Gly Phe Val Asn
           10                  15                  20
   Ala Gly Leu Arg Ser Glu Tyr Asn His Pro Thr Lys Val His Gly Ser
       25                  30                  35
   Thr Val Gln Lys Leu Ile Asp Gly Lys Val Asp Lys Thr Asn Arg Ser
   40                  45                  50                  55
   Ile Asp Thr Ala Ala Gly Arg Tyr Ser Asn Ala Tyr Val Gly Ala Ile
                   60                  65                  70
   Asn Ser Pro Gly Leu Lys Gly Arg Tyr Tyr Tyr Arg Thr Asn
               75                  80                  85